(19) Europäisches Patentamt
European Patent Office
Office européen des brevets

(11) EP 4 302 776 A1

(12) **EUROPEAN PATENT APPLICATION**
published in accordance with Art. 153(4) EPC

(43) Date of publication:
**10.01.2024 Bulletin 2024/02**

(21) Application number: **21911617.5**

(22) Date of filing: **24.12.2021**

(51) International Patent Classification (IPC):
***A61K 38/26*** *(2006.01)* ***A61K 38/22*** *(2006.01)*
***A61K 47/68*** *(2017.01)* ***A61P 1/00*** *(2006.01)*
***A61P 29/00*** *(2006.01)* ***C07K 14/605*** *(2006.01)*

(52) Cooperative Patent Classification (CPC):
**A61K 38/22; A61K 38/26; A61K 47/68; A61P 1/00; A61P 29/00; C07K 14/605**

(86) International application number:
**PCT/KR2021/019854**

(87) International publication number:
**WO 2022/139552 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.12.2020 KR 20200183440**

(71) Applicant: **Hanmi Pharm. Co., Ltd.**
**Hwaseong-si, Gyeonggi-do 18536 (KR)**

(72) Inventors:
- **CHOI, Jae Hyuk**
  **Hwaseong-si, Gyeonggi-do 18469 (KR)**
- **LEE, Jin Bong**
  **Hwaseong-si, Gyeonggi-do 18469 (KR)**
- **LEE, Sang Hyun**
  **Hwaseong-si, Gyeonggi-do 18469 (KR)**
- **KWON, Hyun Joo**
  **Hwaseong-si, Gyeonggi-do 18469 (KR)**
- **HONG, Sung Hee**
  **Hwaseong-si, Gyeonggi-do 18469 (KR)**
- **YOO, Nyeong Sang**
  **Hwaseong-si, Gyeonggi-do 18469 (KR)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) COMBINATION THERAPY OF INSULINOTROPIC PEPTIDE AND GLP-2, FOR PREVENTING OR TREATING SHORT BOWEL SYNDROME

(57) The present invention relates to a combination therapy using an insulinotropic peptide and GLP-2 for the prevention, improvement, or treatment of short bowel syndrome.

[FIG. 1]

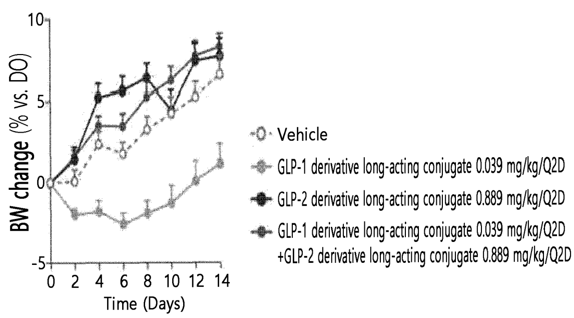


EP 4 302 776 A1

## Description

### [Technical Field]

The present invention relates to a combination therapy using an insulinotropic peptide and GLP-2 for the prevention or treatment of short bowel syndrome.

### [Background Art]

Short bowel syndrome (SBS) is a digestive malabsorption disorder caused by congenital shortness of the small intestine or acquired loss (reduced absorption area of the small intestine) of a certain percentage of the small intestine due to surgery and the like, and the symptoms may include lack of nutrient absorption, malabsorption of nutrients, abdominal pain, diarrhea, steatorrhea, lactose intolerance, dehydration, weight loss, lethargy, fatigue, and the like.

Glucagon-like peptide-2 (GLP-2) is a peptide hormone composed of 33 amino acids produced by L-cells of the small intestine in response to ingested nutrients. GLP-2 induces mucosal growth in the small and large intestine, promotes the growth of enterocytes and crypt cells, and inhibits apoptosis. GLP-2 also increases the absorption of nutrients in the small intestine and reduces intestinal permeability. GLP-2 inhibits gastric emptying and secretion of gastric acid, increases intestinal blood flow rate, and relaxes intestinal smooth muscle. It has also been reported that GLP-2 enhances nutrient absorption and absorption in the digestive tract in rodent experimental models with short bowel syndrome (Ljungmann K. et al., Am. J. Physiol. Gastrointest Liver Physiol., 2001, 281(3):G779-85).

Meanwhile, glucagon-like peptide-1 (GLP-1), a type of insulinotropic peptide, is an incretin hormone secreted from L-cells of the ileum and colon. The main action of GLP-1 is to increase insulin secretion, and glucose-dependent secretion is made so that hypoglycemia does not occur. GLP-1 is applied as a treatment method for type 2 diabetes because of the characteristics, but is significantly limited in its development as a drug since its half-life in blood is as short as around 2 minutes. Exendin-4, developed and marketed as a GLP-1 agonist, is a GLP-1 analog purified from the salivary glands of the Gila monster. Exendin-4 has resistance to DPP-IV (dipeptidyl peptidase-4) as well as higher physiological activity than GLP-1, and thus has an *in vivo* half-life of 2 to 4 hours, which is longer than that of GLP-1 (US 5,424,286). However, sufficient duration of physiological activity cannot be expected only by increasing the resistance to DPP-IV. For example, the exendin-4 that is currently on the market (exenatide) is required to be administered to patients twice a day by injection, and still has a disadvantage in that induction of vomiting and nausea caused by administration are greatly burdensome to patients.

Thus far, the development of therapeutic agents for short bowel syndrome using GLP-2 has been incomplete, and therefore there is a need for continuing development of therapeutic agents.

### [Disclosure]

### [Technical Problem]

An object of the present invention is to provide a therapy for the prevention, improvement, or treatment of short bowel syndrome using an insulinotropic peptide and glucagon-like peptide-2 (GLP-2) in combination.

Another object of the present invention is to provide a pharmaceutical composition for the prevention or treatment of short bowel syndrome containing an insulinotropic peptide, in which the pharmaceutical composition is administered in combination with GLP-2.

Another object of the present invention is to provide a pharmaceutical composition for the prevention or treatment of short bowel syndrome containing GLP-2, in which the pharmaceutical composition is administered in combination with an insulinotropic peptide.

Another object of the present invention is to provide a combination containing an insulinotropic peptide and GLP-2.

Another object of the present invention is to provide a pharmaceutical composition for the prevention, improvement, or treatment of short bowel syndrome, containing the combination.

Another object of the present invention is to provide a pharmaceutical kit for the prevention, improvement, or treatment of short bowel syndrome, containing an insulinotropic peptide and GLP-2.

Another object of the present invention is to provide a method for preventing, improving, or treating short bowel syndrome, which includes administering and/or using the combination, pharmaceutical compositions, or pharmaceutical kit in a subject in need thereof.

Another object of the present invention is to provide a method for preventing, improving, or treating short bowel syndrome, which includes administering and/or using in combination with a composition containing an insulinotropic peptide in a pharmaceutically effective amount and a composition containing GLP-2 in a pharmaceutically effective

amount in a subject in need thereof.

Another object of the present invention is to provide use of the combination, pharmaceutical compositions, or pharmaceutical kit for the prevention, improvement, or treatment of short bowel syndrome and/or use of the combination, pharmaceutical compositions, or pharmaceutical kit for the preparation of a medicament for the prevention, improvement, or treatment of short bowel syndrome.

**[Technical Solution]**

An aspect embodying the present invention is a therapy for the prevention, improvement, or treatment of short bowel syndrome using an insulinotropic peptide and GLP-2 in combination.

An aspect embodying the present invention is a composition for the prevention, improvement, or treatment of short bowel syndrome containing an insulinotropic peptide, in which the insulinotropic peptide is administered in combination with GLP-2.

As an embodiment, the present invention is a pharmaceutical composition for the prevention, improvement, or treatment of short bowel syndrome containing an insulinotropic peptide in a pharmaceutically effective amount, in which the pharmaceutical composition is administered in combination with GLP-2.

As another embodiment, the present invention is a food composition for the prevention or improvement of short bowel syndrome containing an insulinotropic peptide, in which the food composition is administered in combination with GLP-2.

An aspect embodying the present invention is a composition for the prevention, improvement, or treatment of short bowel syndrome containing GLP-2, in which GLP-2 is administered in combination with an insulinotropic peptide.

As an embodiment, the present invention is a pharmaceutical composition for the prevention, improvement, or treatment of short bowel syndrome containing GLP-2 in a pharmaceutically effective amount, in which the pharmaceutical composition is administered in combination with an insulinotropic peptide.

As another embodiment, the present invention is a food composition for the prevention or improvement of short bowel syndrome containing GLP-2, in which the food composition is administered in combination with an insulinotropic peptide.

The composition according to any one of the preceding embodiments, in which the insulinotropic peptide is selected from the group consisting of glucagon-like peptide-1 (GLP-1); exendin-3; exendin-4; agonists, derivatives, fragments, and variants of the glucagon-like peptide-1 (GLP-1), exendin-3, and exendin-4; and combinations thereof.

The composition according to any one of the preceding embodiments, in which the insulinotropic peptide is an insulinotropic peptide derivative in which a N-terminal histidine residue of an insulinotropic peptide is substituted with imidazoacetyldeshistidine, desaminohistidine, β-hydroxyimidazopropionyldeshistidine, *N*-dimethylhistidine, or β-carboxyimidazopropionyldeshistidine.

The composition according to any one of the preceding embodiments, in which the insulinotropic peptide is native exendin-4, an exendin-4 derivative in which an *alpha*-carbon of a histidine residue, which is a first amino acid at a N-terminus of exendin-4, and a N-terminal amino group bound to the *alpha*-carbon are removed, an exendin-4 derivative in which a N-terminal amino group of exendin-4 is removed, an exendin-4 derivative in which a N-terminal amino group of exendin-4 is substituted with a hydroxyl group, an exendin-4 derivative in which a N-terminal amino group of exendin-4 is modified with two methyl groups, an exendin-4 derivative in which a N-terminal amino group of exendin-4 is substituted with a carboxyl group, an exendin-4 derivative in which a twelfth amino acid (lysine) of exendin-4 is substituted with serine, or an exendin-4 derivative in which a twelfth amino acid (lysine) of exendin-4 is substituted with arginine.

The composition according to any one of the preceding embodiments, in which the GLP-2 is native GLP-2 or a GLP-2 derivative.

The composition according to any one of the preceding embodiments, in which the GLP-2 derivative is a GLP-2 derivative in which at least one amino acid in a native GLP-2 sequence is subjected to variation selected from the group consisting of substitution, addition, deletion, modification, and a combination thereof.

The composition according to any one of the preceding embodiments, in which the GLP-2 derivative is subjected to variation of at least one amino acid among amino acids 1, 2, 30, and 33 in SEQ ID NO: 1.

The composition according to any one of the preceding embodiments, in which the GLP-2 derivative includes an amino acid sequence represented by the following Formula 1:

[Formula 1] $X_1X_2$DGSFSDEMNTILDNLAARDFINWLIQT$X_{30}$ITD$X_{34}$ (SEQ ID NO: 9)

wherein,

$X_1$ is histidine, imidazoacetyldeshistidine, desaminohistidine, β-hydroxyimidazopropionyldeshistidine, *N*-dimethylhistidine, or β-carboxyimidazopropionyldeshistidine;

$X_2$ is alanine, glycine, or 2-aminoisobutyric acid (Aib);
$X_{30}$ is lysine or arginine; and
$X_{34}$ is absent or is lysine, arginine, glutamine, histidine, 6-azidolysine, or cysteine;
provided that the sequence of SEQ ID NO: 1 is excluded from the amino acid sequences represented by Formula 1.

The composition according to any one of the preceding embodiments, in which in the GLP-2 derivative,

(1) $X_1$ is imidazoacetyldeshistidine, $X_2$ is glycine, $X_{30}$ is lysine, and $X_{34}$ is cysteine;
(2) $X_1$ is imidazoacetyldeshistidine, $X_2$ is glycine, $X_{30}$ is lysine, and $X_{34}$ is lysine;
(3) $X_1$ is imidazoacetyldeshistidine, $X_2$ is glycine, $X_{30}$ is arginine, and $X_{34}$ is lysine;
(4) $X_1$ is imidazoacetyldeshistidine, $X_2$ is glycine, $X_{30}$ is lysine, and $X_{34}$ is 6-azidolysine;
(5) $X_1$ is imidazoacetyldeshistidine, $X_2$ is glycine, $X_{30}$ is arginine, and $X_{34}$ is cysteine;
(6) $X_1$ is imidazoacetyldeshistidine, $X_2$ is Aib, $X_{30}$ is lysine, and $X_{34}$ is cysteine; or
(7) $X_1$ is histidine, $X_2$ is Aib, $X_{30}$ is lysine, and $X_{34}$ is cysteine.

The composition according to any one of the preceding embodiments, in which the GLP-2 derivative includes an amino acid sequence represented by the following Formula 2:

[Formula 2] $X_1X_2$DGSFSDEMNTILDNLAARDFINWLIQT$X_{30}$ITD$X_{34}$ (SEQ ID NO: 10)

wherein,

$X_1$ is histidine, imidazoacetyldeshistidine, desaminohistidine, β-hydroxyimidazopropionyldeshistidine, N-dimethylhistidine, or β-carboxyimidazopropionyldeshistidine;
$X_2$ is alanine, glycine, or 2-aminoisobutyric acid (Aib);
$X_{30}$ is lysine or arginine; and
$X_{34}$ is one or more arbitrary amino acids or one or more arbitrary amino acids subjected to variation;
provided that the sequence of SEQ ID NO: 1 is excluded from the amino acid sequences represented by Formula 2.

The composition according to any one of the preceding embodiments, in which the GLP-2 derivative is a peptide having an amino acid sequence selected from the group consisting of SEQ ID NOs: 2 to 8.

The composition according to any one of the preceding embodiments, in which the composition, when administered to a subject, causes one or more among weight gain, an increase in small intestine length, a decrease in gastrointestinal motility, and an increase in nutrient absorption.

The composition according to any one of the preceding embodiments, in which the insulinotropic peptide and GLP-2 have a C-terminus unvaried or amidated.

The composition according to any one of the preceding embodiments, in which (i) the insulinotropic peptide is in a form of a long-acting conjugate to which a biocompatible substance capable of increasing *in vivo* half-life of the insulinotropic peptide is bound,

(ii) the GLP-2 is in a form of a long-acting conjugate to which a biocompatible substance capable of increasing *in vivo* half-life of the GLP-2 is bound; or
(iii) each of the insulinotropic peptide and GLP-2 is in a form of a long-acting conjugate to which a biocompatible substance capable of increasing *in vivo* half-life of each of the insulinotropic peptide and GLP-2 is bound.

The composition according to any one of the preceding embodiments, in which the conjugate is represented by the following Chemical Formula 1 :

[Chem. 1] $X\text{-}L_a\text{-}F$

wherein,

X is an insulinotropic peptide or GLP-2;
L is a linker containing an ethylene glycol repeating unit;
a is 0 or a natural number, provided that each L is independent of each other when a is 2 or more;
F is an immunoglobulin Fc region; and
the "-" is a covalent bond.

The composition according to any one of the preceding embodiments, in which the immunoglobulin Fc region is an aglycosylated IgG4 Fc region.

The composition according to any one of the preceding embodiments, in which the F is a dimer composed of two polypeptide chains, and one end of L is linked only to one polypeptide chain of the two polypeptide chains.

The composition according to any one of the preceding embodiments, in which the L is polyethylene glycol.

The composition according to any one of the preceding embodiments, in which a chemical formula weight of an ethylene glycol repeating unit moiety in the L is in a range of 1 kDa to 100 kDa.

The composition according to any one of the preceding embodiments, which further contains a pharmaceutically acceptable carrier, excipient, or diluent.

The composition according to any one of the preceding embodiments, in which the composition containing an insulinotropic peptide is administered in combination with the composition containing GLP-2 simultaneously, sequentially, or in reverse order.

Another aspect embodying the present invention is a combination containing an insulinotropic peptide and GLP-2.

Another aspect embodying the present invention is a pharmaceutical composition for the prevention, improvement, or treatment of short bowel syndrome, containing the combination.

Another aspect embodying the present invention is a pharmaceutical kit for the prevention, improvement, or treatment of short bowel syndrome, containing the combination.

Another aspect embodying the present invention is a method for preventing, improving, or treating short bowel syndrome, which includes administering and/or using the combination, pharmaceutical compositions, or pharmaceutical kit in a subject in need thereof.

Another aspect embodying the present invention is a method for preventing, improving, or treating short bowel syndrome, which includes administering and/or using in combination with a composition containing an insulinotropic peptide in a pharmaceutically effective amount and a composition containing GLP-2 in a pharmaceutically effective amount in a subject in need thereof.

Another aspect embodying the present invention is use of the combination, pharmaceutical compositions, or pharmaceutical kit for the prevention, improvement, or treatment of short bowel syndrome and/or use of the combination, pharmaceutical compositions, or pharmaceutical kit for the preparation of a medicament for the prevention or treatment of short bowel syndrome.

## [Advantageous Effects]

The therapy by combined administration of an insulinotropic peptide and GLP-2 according to the present invention exhibits an improved effect compared to the therapy by single administration, and can be thus usefully used to prevent, improve, or treat short bowel syndrome.

## [Brief Description of Drawings]

FIG. 1 is a diagram illustrating changes in mouse weight by combined administration of an insulinotropic peptide (GLP-1 derivative long-acting conjugate) and GLP-2 (GLP-2 derivative long-acting conjugate);

FIG. 2 is a diagram illustrating changes in length of the small intestine of mice by combined administration of an insulinotropic peptide (GLP-1 derivative long-acting conjugate) and GLP-2 (GLP-2 derivative long-acting conjugate); and

FIG. 3 is a diagram illustrating changes in gastrointestinal motility by combined administration of an insulinotropic peptide (GLP-1 derivative long-acting conjugate) and GLP-2 (GLP-2 derivative long-acting conjugate).

## [Detailed Description of the Invention]

Hereinafter, the present invention will be described in more detail.

Meanwhile, the description and embodiment disclosed herein may also be applied to other descriptions and other embodiments, respectively. In other words, all combinations of the various elements disclosed herein fall within the scope of the present invention. In addition, it cannot be said that the scope of the present invention is limited by the specific description described below.

In addition, those skilled in the art will recognize, or be able to ascertain using no more than routine experimentation, many equivalents to the specific aspects of the present invention described in this application. In addition, such equivalents are intended to be included in the present invention.

Throughout this specification, conventional one-letter and three-letter codes for naturally occurring amino acids are used as well as generally accepted three letter codes for other amino acids such as 2-aminoisobutyric acid (Aib) and 6-azidolysine ($_{AZ}$K) are used. In addition, amino acids referred to by abbreviations herein are described according to the IUPAC-IUB nomenclature.

Alanine Ala, A
Arginine Arg, R
Asparagine Asn, N
Aspartic acid Asp, D
Cysteine Cys, C
Glutamic acid Glu, E
Glutamine Gln, Q
Glycine Gly, G
Histidine His, H
Isoleucine Ile, I
Leucine Leu, L
Lysine Lys, K
Methionine Met, M
Phenylalanine Phe, F
Proline Pro, P
Serine Ser, S
Threonine Thr, T
Tryptophan Trp, W
Tyrosine Tyr, Y
Valine Val, V

An aspect embodying the present invention provides a pharmaceutical composition for the prevention, improvement, or treatment of short bowel syndrome (SBS) containing an insulinotropic peptide, in which the insulinotropic peptide is administered in combination with glucagon-like peptide-2 (GLP-2).

In an embodiment of the present invention, the pharmaceutical composition may be a pharmaceutical composition for the prevention or treatment of short bowel syndrome containing an insulinotropic peptide in a pharmaceutically effective amount, in which the pharmaceutical composition is administered in combination with GLP-2 (for example, a pharmaceutically effective amount of GLP-2), but is not limited thereto.

Another aspect embodying the present invention provides a pharmaceutical composition for the prevention, improvement, or treatment of short bowel syndrome containing GLP-2, in which GLP-2 is administered in combination with an insulinotropic peptide.

In an embodiment of the present invention, the pharmaceutical composition may be a pharmaceutical composition for the prevention, improvement, or treatment of short bowel syndrome containing GLP-2 in a pharmaceutically effective amount, in which the pharmaceutical composition is administered in combination with an insulinotropic peptide (for example, a pharmaceutically effective amount of insulinotropic peptide), but is not limited thereto.

In an embodiment of the present invention, the combined administration may be (i) combined administration using a pharmaceutical composition further containing GLP-2 in the form of a mixture or in a separate form together with an insulinotropic peptide, or (ii) simultaneous, sequential or reverse order combined administration of the pharmaceutical composition containing an insulinotropic peptide with a separate pharmaceutical composition containing GLP-2, but is not limited thereto.

Another aspect of the present invention provides use of an insulinotropic peptide and GLP-2 in combination for the prevention, improvement, or treatment of short bowel syndrome.

Another embodiment provides a pharmaceutical composition for the prevention, improvement, or treatment of short bowel syndrome, using an insulinotropic peptide and GLP-2 in combination.

Specifically, an aspect of the present invention provides a combination, pharmaceutical composition, or pharmaceutical kit containing an insulinotropic peptide and GLP-2. An embodiment provides a combination, pharmaceutical composition, or pharmaceutical kit for the prevention, improvement, or treatment of short bowel syndrome.

In the present invention, the term "combination" refers to the use of an insulinotropic peptide and GLP-2 for combined administration, and may be understood as the same meaning as "combined used". This also includes, but is not limited to, the forms of a pharmaceutical composition and a pharmaceutical kit in which an insulinotropic peptide and GLP-2 are used in combination.

The combination may be

a) one in which an insulinotropic peptide and GLP-2 are administered as a mixture; or
b) one in which an insulinotropic peptide and GLP-2 are administered in separate forms, but is not limited thereto.

In a case where an insulinotropic peptide and GLP-2 are in separate forms, each of the insulinotropic peptide and GLP-2 may be prepared as separate preparations, and the separate preparations may be administered simultaneously, separately, sequentially, or in reverse order.

In the present invention, "combined administration", "used in combination", or "using in combination" means not only simultaneous administration, but is also to be understood as an administration form in which the insulinotropic peptide and GLP-2 act together on a subject so that each substance can perform a function at a level equal to or higher than its original function. Accordingly, when the term "use in combination" is used herein, this is to be understood that the use in combination refers to simultaneous, separate, sequential, or reverse order administration, and the order is unlimited. When the administration is sequential, reverse order, or separate, the order of administration is not particularly limited, but the administration interval of the second ingredient is to be such that the beneficial effect by the use in combination is not lost.

In the present invention, the term "composition containing a combination" may be the combination itself containing an insulinotropic peptide and GLP-2, or may contain the combination and have a therapeutic use, but is not limited thereto. For example, the composition may have use for the prevention, improvement, or treatment of short bowel syndrome, but is not limited thereto. As used herein, a "composition containing a combination" may be used interchangeably with a "composition".

The composition containing a combination according to the present invention is for combined administration of an insulinotropic peptide and GLP-2, and the insulinotropic peptide and GLP-2 may be prepared as one preparation or may be prepared separately. Specifically, the composition may be one in which an insulinotropic peptide and GLP-2 are administered simultaneously, separately, sequentially, or in reverse order, but is not limited thereto.

In the present invention, the term "kit" may include a combination or composition according to the present invention in order to administer an insulinotropic peptide and GLP-2 in combination. Specifically, the kit according to the present invention may contain an insulinotropic peptide and GLP-2 that are prepared as one preparation; or may contain separate preparations of an insulinotropic peptide and GLP-2, and may additionally contain substances required for combined administration of the two substances, but is not limited thereto.

The present invention has confirmed that the effect of preventing, improving, or treating short bowel syndrome is dramatically enhanced in the case of using an insulinotropic peptide in combination with GLP-2 compared to that in the case of using an insulinotropic peptide singly, and thus provides the combination therapy.

The "insulinotropic peptide" refers to a peptide having an insulinotropic function, and can stimulate the synthesis or expression of insulin in pancreatic beta cells. The insulinotropic peptide may be, for example, glucagon-like peptide-1 (GLP-1), exendin-3 or exendin-4, but is not limited thereto. The insulinotropic peptide includes not only a native insulinotropic peptide, but also precursors, agonists, derivatives, fragments, and variants thereof, and also include long-acting conjugates to which biocompatible substances capable of increasing their *in vivo* half-life are bound. The insulinotropic peptide may be contained in the pharmaceutical composition in a pharmaceutically effective amount.

GLP-1 is a hormone secreted from the small intestine and generally promotes insulin biosynthesis and secretion, inhibits glucagon secretion, and promotes intracellular glucose uptake. In the small intestine, glucagon precursors are broken down into three peptides: glucagon, GLP-1, and GLP-2. Here, GLP-1 means GLP-1 (1-37), which is a form without having an insulinotropic function, and is processed into a GLP-1 (7-37) form to become active GLP-1 (7-37). The amino acid sequence of GLP-1 (7-37) is as follows.
GLP-1 (7-37):
HAEGT FTSDV SSYLE GQAAK EFIAW LVKGR G (SEQ ID NO: 32)

Exendin-3 and exendin-4 are GLP-1 analogs or derivatives, which consist of 39 amino acids, have 53% amino acid sequence similarity with GLP-1, and correspond to insulinotropic peptides. The amino acid sequences of exendin-3 and exendin-4 are as follows.

Exendin-3:
HSDGT FTSDL SKQME EEAVR LFIEW LKNGG PSSGA PPPS (SEQ ID NO: 33)
Exendin-4:
HGEGT FTSDL SKQME EEAVR LFIEW LKNGG PSSGA PPPS (SEQ ID NO: 34)

The insulinotropic peptide derivative may have an insulinotropic function and have at least 80%, 85%, 90%, 95%, 96%, 97%, 98%, or 99% or more homology or identity to the native insulinotropic peptide in amino acid sequence, and/or may be in a form in which some groups of amino acid residues of the insulinotropic peptide may be chemically substituted (for example, *alpha*-methylation, *alpha*-hydroxylation), removed (for example, deamination) or modified (for example, *N*-methylation), but is limited thereto.

In a specific embodiment, the insulinotropic peptide derivative of the present invention may be prepared by a method in which the alpha amino group of N-terminal histidine is removed, a method in which the N-terminal amino group is substituted with a hydroxyl group or a carboxyl group and synthesis is performed, a method in which the *alpha*-carbon of N-terminal histidine and the N-terminal amino group bound to the *alpha*-carbon are removed to leave only the imidazo-acetyl functional group, a method in which the N-terminal amino group is modified with two methyl groups, and the like. The insulinotropic peptide derivative prepared through the methods may be an insulinotropic peptide derivative in which the N-terminal histidine residue of an insulinotropic peptide is substituted with imidazoacetyldeshistidine, desaminohistidine, β-hydroxyimidazopropionyldeshistidine, *N*-dimethylhistidine, or β-carboxyimidazopropionyldeshistidine.

As a specific example, the insulinotropic peptide derivative may be a GLP-1 derivative, and the GLP-1 derivative includes, but are not limited to, exendin-3, exendin-4, or derivatives thereof.

In addition, an example of the insulinotropic peptide derivative may be a derivative obtained by chemically varying the N-terminal amino group or amino acid residue of exendin-4, and may be imidazoacetyl-deshistidyl-exendin-4 (CA-exendin-4) in which the *alpha*-carbon of the histidine residue, which is the first N-terminal amino acid of exendin-4, and the N-terminal amino group bound to the *alpha*-carbon are removed; desaminohistidyl exendin-4 (DA-exendin-4) in which the N-terminal amino group of exendin-4 is removed; β-hydroxyimidazopropionyldeshistidyl exendin-4 (HY-exendin-4) in which the N-terminal amino group of exendin-4 is substituted with a hydroxyl group; *N*-dimethylhistidyl exendin-4 (DM-exendin-4) in which the N-terminal amino group of exendin-4 is modified with two methyl groups; or β-carboxyimidazopropionyl-deshistidyl exendin-4 (CX-exendin-4) in which the N-terminal amino group of exendin-4 is substituted with a carboxyl group; an exendin-4 derivative in which the twelfth amino acid (lysine) of exendin-4 is substituted with serine; or an exendin-4 derivative in which the twelfth amino acid (lysine) of exendin-4 is substituted with arginine, but is not limited thereto.

The insulinotropic peptide fragment refers to a form in which one or more amino acids are removed from and/or added to the N-terminus or C-terminus of the native insulinotropic peptide, and the added amino acid may also be an amino acid that does not exist in nature (for example, D-type amino acid).

The insulinotropic peptide variant refers to a peptide that differs from the native insulinotropic peptide by one or more amino acids, exhibits at least 80%, 85%, 90%, 95%, 96%, 97%, 98%, or 99% or more homology or identity to the native insulinotropic peptide, and possesses an insulinotropic function.

The insulinotropic peptide agonist refers to a substance that exhibits the same biological activity as the insulinotropic peptide by binding to an *in vivo* receptor of the insulinotropic peptide regardless of the structure of the insulinotropic peptide.

The preparation methods used for the derivative, fragment, variant, and agonist of the present invention, respectively may be used independently or in combination. For example, insulinotropic peptides having one or more different amino acid sequences and deamination at the N-terminal amino acid residue are also included in the present invention.

In the present invention, the term "glucagon-like peptide-2" or "GLP-2" is an agonist of the glucagon-like peptide-2 receptor, and may be in the form of a polypeptide or in the form of a long-acting conjugate to which a biocompatible substance capable of increasing the *in vivo* half-life of the polypeptide is bound, but is not limited thereto. In the present invention, when referring to glucagon-like peptide-2 or GLP-2, not only sequences identical to native human GLP-2 but also GLP-2 derivatives are included, and glucagon-like peptide-2 or GLP-2 also covers long-acting conjugates in which biocompatible substances are bound to native GLP-2 or GLP-2 derivatives. The GLP-2 may be contained in the composition or in a separate composition in a pharmaceutically effective amount so as to be administered or used in combination with a pharmaceutical composition containing an insulinotropic peptide.

The amino acid sequence of native GLP-2 is as follows.

GLP-2 (1-33):

HADGSFSDEMNTILDNLAARDFINWLIQTKITD (SEQ ID NO: 1)

In the present invention, "agonist of GLP-2 receptor" refers to a substance that binds to an *in vivo* or isolated human glucagon-like peptide-2 receptor to exhibit physiological activity equivalent to or similar to that of native GLP-2. For example, the GLP-2 agonist may include native GLP-2 or GLP-2 derivatives.

In the present invention, "GLP-2 derivative" includes a peptide having one or more differences in amino acid sequence compared to native GLP-2; a peptide varied through modification of the native GLP-2 sequence; and/or a mimic of native GLP-2 that, like native GLP-2, has functions for preventing, treating, and/or improving short bowel syndrome. Specifically, the GLP-2 derivative may be one in which at least one or more amino acids in the native GLP-2 sequence are subjected to variation selected from the group consisting of substitution, addition, deletion, modification, and combinations thereof, but is not limited thereto.

The added amino acid can also be a non-native amino acid (for example, D-type amino acid), and substitution with a non-native amino acid in addition to a native amino acid is also possible. The added amino acid sequence may be derived from native GLP-2, but is not limited thereto. In addition, in the present invention, variation of amino acids

may mean that some groups of amino acid residues are chemically substituted (for example, *alpha*-methylation, *alpha*-hydroxylation, substitution with an azido group), removed (for example, deamination), and/or modified (for example, *N*-methylation) together with; or independently of the substitution, addition, or removal of at least one amino acid or combinations thereof, but is not limited thereto.

In a specific embodiment, the GLP-2 derivative of the present invention may have at least 80%, 85%, 90%, 95%, 96%, 97%, 98%, or 99% or more homology or identity to native GLP-2 in amino acid sequence, and/or may be in a form in which some groups of an amino acid residue in GLP-2 are chemically substituted (for example, *alpha*-methylation, *alpha*-hydroxylation, substitution with an azido group), removed (for example, deamination), and/or modified (for example, *N*-methylation), but is not limited thereto.

In a specific embodiment, the N-terminal amino group of GLP-2 of the present invention may be substituted, removed, or modified, but is not limited thereto. In order to prevent binding to the N-terminus, which is an important site for the *in vivo* activity of GLP-2, when a long-acting conjugate is prepared, GLP-2 of the present invention may be prepared by a method in which the alpha amino group of the N-terminal histidine is removed, a method in which the N-terminal amino group is substituted with a hydroxyl group or a carboxyl group and synthesis is performed, a method in which the *alpha*-carbon of the N-terminal histidine and the N-terminal amino group bound to the *alpha*-carbon are removed to leave only the imidazo-acetyl functional group, a method in which the N-terminal amino group is modified with two methyl groups, and the like.

Specifically, the GLP-2 derivative may be imidazoacetyl-deshistidyl-GLP-2 (CA-GLP-2) in which the *alpha*-carbon of the histidine residue, which is the first amino acid at the N-terminus of GLP-2, and the N-terminal amino group bound to the *alpha*-carbon are removed; desaminohistidyl GLP-2 (DA-GLP-2) in which the N-terminal amino group of GLP-2 is removed; β-hydroxyimidazopropionyldeshistidyl GLP-2 (HY-GLP-2) in which the N-terminal amino group of GLP-2 is substituted with a hydroxyl group; *N*-dimethylhistidyl GLP-2 (DM-GLP-2) in which the N-terminal amino group of GLP-2 is modified with two methyl groups; or β-carboxyimidazopropionyl-deshistidyl GLP-2 (CX-GLP-2) in which the N-terminal amino group of GLP-2 is substituted with a carboxyl group, but is not limited thereto. As a non-limiting example, the structures of substances used to prepare the GLP-2 derivatives are as follows.

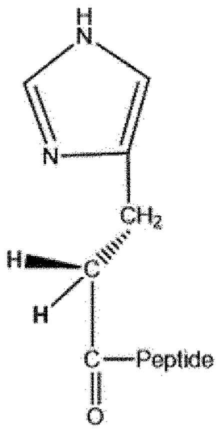

**Des-amino-histidyl (DA)-GLP-2**

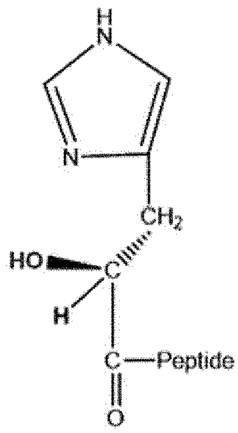

**Beta-hydroxy-imidazopropionyl (HY)-GLP-2**

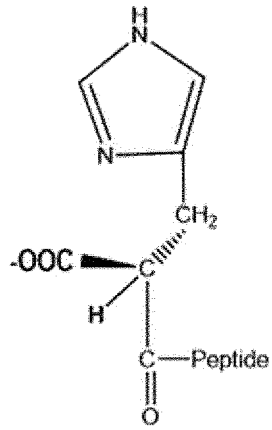

**Beta-carboxyl-imidazopropionyl (CX)-GLP-2**

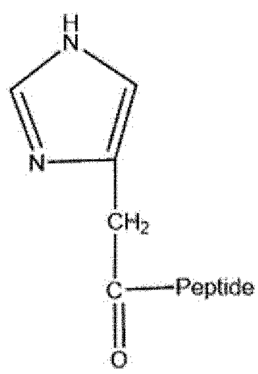 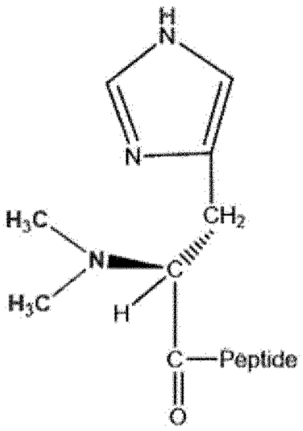

**Imidazoacetyl (CA)-GLP-2** **Dimethyl-histidyl (DM)-GLP-2**

Herein, imidazoacetyldeshistidyl (dine), desaminohistidyl (dine), β-hydroxyimidazopropionyldeshistidyl (dine), *N*-dimethylhistidyl (dine), and β-carboxyimidazopropionyldeshistidyl (dine) may be used in the same meaning as imidazoacetyl, des-amino-histidyl, β-hydroxy-imidazopropionyl, dimethyl-histidyl, and β-carboxyl-imidazopropionyl, respectively.

In a specific embodiment, the GLP-2 derivative may be subjected to variation of at least one amino acid among amino acids 1, 2, 30, and 33 in SEQ ID NO: 1, but is not limited thereto. Specifically, the variation may be variation selected from the group consisting of substitution, addition, removal, and modification of at least one amino acid, and combinations thereof, the amino acid added at this time can also be a non-native amino acid (for example, D-type amino acid), and substitution with a non-native amino acid in addition to a native amino acid is also possible. The added amino acid sequence may be derived from native GLP-2, but is not limited thereto. In addition, in the present invention, variation of amino acids may mean that some groups of amino acid residues are chemically substituted (for example, *alpha*-methylation, *alpha*-hydroxylation, substitution with an azido group), removed (for example, deamination), and/or modified (for example, *N*-methylation) together with; or independently of the substitution, addition, or removal of at least one amino acid or combinations thereof, but is not limited thereto.

In a specific embodiment, the GLP-2 derivative may include, but is not limited to, an amino acid sequence represented by the following Formula 1:

[Formula 1] $X_1X_2$DGSFSDEMNTILDNLAARDFINWLIQT$X_{30}$ITD$X_{34}$ (SEQ ID NO: 9)

wherein,

- $X_1$ is histidine, imidazoacetyldeshistidine, desaminohistidine, β-hydroxyimidazopropionyldeshistidine, *N*-dimethylhistidine, or β-carboxyimidazopropionyldeshistidine;
- $X_2$ is alanine, glycine, or 2-aminoisobutyric acid (Aib);
- $X_{30}$ is lysine or arginine; and
- $X_{34}$ is absent or is lysine, arginine, glutamine, histidine, 6-azidolysine, or cysteine.

In another specific embodiment, the GLP-2 derivative may include, but is not limited to, an amino acid sequence represented by the following Formula 2:

[Formula 2] $X_1X_2$DGSFSDEMNTILDNLAARDFINWLIQT$X_{30}$ITD$X_{34}$ (SEQ ID NO: 10)

wherein,

- $X_1$ is histidine, imidazoacetyldeshistidine, desaminohistidine, β-hydroxyimidazopropionyldeshistidine, *N*-dimethylhistidine, or β-carboxyim idazopropionyldeshistidine;
- $X_2$ is alanine, glycine, or 2-aminoisobutyric acid (Aib);
- $X_{30}$ is lysine or arginine; and
- $X_{34}$ is one or more arbitrary amino acids or one or more arbitrary amino acids subjected to variation.

Specifically, the amino acid may be a native amino acid or a non-native amino acid, and the variation of amino acid is as described above.

In addition, among the amino acid sequences represented by Formula 1 or 2, the same sequence as SEQ ID NO: 1 may be excluded from GLP-2 derivatives, but the GLP-2 derivative is not limited thereto.

Specifically, the GLP-2 derivative of the present invention may have substitution of alanine, the second amino acid of native GLP-2, with glycine or Aib (2-aminoisobutyric acid); substitution of lysine, the 30th amino acid, with arginine; or a combination thereof, but is not limited thereto. In addition, in the GLP-2 derivative, a thiol group (for example, cysteine), an amino group (for example, lysine, arginine, glutamine or histidine), or an azide group (for example, 6-azidolysine) may be introduced into the C-terminus (for example, 33rd amino acid), but the GLP-2 derivative is not limited thereto.

When a long-acting conjugate of a GLP-2 derivative is prepared, since bonding occurs at the introduced group, a GLP-2 conjugate having a selectively controlled binding position may be prepared using this. Specifically, one end of the linker may be bound to the hydroxyl group, thiol group, amino group or azide group of the GLP-2 derivative, and a substance capable of increasing the *in vivo* half-life (for example, an immunoglobulin Fc region) may be bound to the other end of the linker. The thiol group, amino group or azide group may be introduced by adding an amino acid to GLP-2, but the introduction is not limited thereto. The thiol group may be introduced by adding cysteine (C) to GLP-2; the amino group may be introduced by adding lysine (K), arginine (R), glutamine (Q) or histidine (H) to GLP-2; and the azide group may be introduced by adding 6-azidolysine ($_{AZ}$K) to GLP-2, but the introduction is not limited thereto.

Specifically, in the GLP-2 derivative according to the present invention, at least one residue may be cysteine, lysine, arginine, glutamine, histidine or 6-azidolysine, but is not limited thereto.

Specifically, the GLP-2 derivative of the present invention includes substitution of alanine, the second amino acid of native GLP-2, with glycine and introduction of a thiol group (for example, cysteine) into the C-terminus, more specifically may include imidazoacetyldeshistidine in which the *alpha*-carbon of the histidine residue, the first N-terminal amino acid, and the N-terminal amino group bound to the *alpha*-carbon are removed, and may have, for example, the amino acid sequence of SEQ ID NO: 2, but is not limited thereto.

Specifically, the GLP-2 derivative of the present invention includes substitution of alanine, the second amino acid of native GLP-2, with glycine and introduction of an amino group (for example, lysine) into the C-terminus, more specifically may include imidazoacetyldeshistidine in which the *alpha*-carbon of the histidine residue, the first N-terminal amino acid, and the N-terminal amino group bound to the *alpha*-carbon are removed, and may have, for example, the amino acid sequence of SEQ ID NO: 3, but is not limited thereto.

Specifically, the GLP-2 derivative of the present invention includes substitution of alanine, the second amino acid of native GLP-2, with glycine, substitution of lysine, the 30th amino acid of native GLP-2, with arginine, and introduction of an amino group (for example, lysine) into the C-terminus, more specifically may include imidazoacetyldeshistidine in which the *alpha*-carbon of the histidine residue, the first N-terminal amino acid, and the N-terminal amino group bound to the *alpha*-carbon are removed, and may have, for example, the amino acid sequence of SEQ ID NO: 4, but is not limited thereto.

Specifically, the GLP-2 derivative of the present invention includes substitution of alanine, the second amino acid of native GLP-2, with glycine and introduction of an azide group (for example, 6-azidolysine) into the C-terminus, more specifically may include imidazoacetyldeshistidine in which the *alpha*-carbon of the histidine residue, the first N-terminal amino acid, and the N-terminal amino group bound to the *alpha*-carbon are removed, and may have, for example, the amino acid sequence of SEQ ID NO: 5, but is not limited thereto.

Specifically, the GLP-2 derivative of the present invention includes substitution of alanine, the second amino acid of native GLP-2, with glycine, substitution of lysine, the 30th amino acid of native GLP-2, with arginine, and introduction of a thiol group (for example, cysteine) into the C-terminus, more specifically may include imidazoacetyldeshistidine in which the *alpha*-carbon of the histidine residue, the first N-terminal amino acid, and the N-terminal amino group bound to the *alpha*-carbon are removed, and may have, for example, the amino acid sequence of SEQ ID NO: 6, but is not limited thereto.

Specifically, the GLP-2 derivative of the present invention includes substitution of alanine, the second amino acid of native GLP-2, with 2-aminoisobutyric acid and introduction of a thiol group (for example, cysteine) into the C-terminus, and may have, for example, the amino acid sequence of SEQ ID NO: 8, and more specifically, the GLP-2 derivative may include imidazoacetyldeshistidine in which the *alpha*-carbon of the histidine residue, the first N-terminal amino acid, and the N-terminal amino group bound to the *alpha*-carbon are removed, and may have, for example, the amino acid sequence of SEQ ID NO: 7, but is not limited thereto.

The GLP-2 derivatives of SEQ ID NOs: 2 to 8 are shown in Table 1 below.

[Table 1]

| Name | Sequence | SEQ ID NO: |
|---|---|---|
| CA GLP-2 KC | $_{ca}$HGDGSFSDEMNTILDNLAARDFINWLIQTKITDC | 2 |
| CA GLP-2 KK | $_{ca}$HGDGSFSDEMNTILDNLAARDFINWLIQTKITDK | 3 |
| CA GLP-2 RK | $_{ca}$HGDGSFSDEMNTILDNLAARDFINWLIQTRITDK | 4 |
| CA GLP-2 $K_{AZ}K$ | $_{ca}$HGDGSFSDEMNTILDNLAARDFINWLIQTKITD$_{AZ}$K | 5 |
| CA GLP-2 RC | $_{ca}$HGDGSFSDEMNTILDNLAARDFINWLIQTRITDC | 6 |
| CA GLP-2 Aib | $_{ca}$HAibDGSFSDEMNTILDNLAARDFINWLIQTKITDC | 7 |
| GLP-2 Aib | HAibDGSFSDEMNTILDNLAARDFINWLIQTKITDC | 8 |

In Table 1, $_{ca}$H indicates that histidine is substituted with imidazoacetyldeshistidine, Aib indicates 2-aminoisobutyric acid, and $_{AZ}$K indicates 6-azido-L-lysyine.

GLP-2 according to the present invention may be a peptide including the specific sequence described above or a peptide consisting of (or essentially consisting of) the specific sequence described above, but is not limited thereto.

Meanwhile, even if a peptide or GLP-2 'consisting of (composed of) a specific sequence number' is described herein, it does not exclude meaningless sequence additions before and after the amino acid sequence of the corresponding sequence number, or naturally occurring mutations, or silent mutations thereof as long as the peptide or GLP-2 exhibits activity the same as or equivalent to that of a peptide or GLP-2 composed of the amino acid sequence of the corresponding sequence number, and it is obvious that a peptide or GLP-2 having such sequence additions or mutations also falls within the scope of the present application.

Specifically, as the GLP-2 derivative, in Formula 1 or 2, (1) $X_2$ may be glycine or Aib; (2) $X_{30}$ may be lysine or arginine, or (3) $X_2$ may be glycine or Aib and $X_{30}$ may be lysine or arginine, but the GLP-2 derivative is not limited thereto.

Specifically, as the GLP-2 derivative, in Formula 1 or 2,

(1) $X_1$ may be imidazoacetyldeshistidine, $X_2$ may be glycine, $X_{30}$ may be lysine, and $X_{34}$ may be cysteine;
(2) $X_1$ may be imidazoacetyldeshistidine, $X_2$ may be glycine, $X_{30}$ may be lysine, and $X_{34}$ may be lysine;
(3) $X_1$ may be imidazoacetyldeshistidine, $X_2$ may be glycine, $X_{30}$ may be arginine, and $X_{34}$ may be lysine;
(4) $X_1$ may be imidazoacetyldeshistidine, $X_2$ may be glycine, $X_{30}$ may be lysine, and $X_{34}$ may be 6-azidolysine;
(5) $X_1$ may be imidazoacetyldeshistidine, $X_2$ may be glycine, $X_{30}$ may be arginine, and $X_{34}$ may be cysteine;
(6) $X_1$ may be imidazoacetyldeshistidine, $X_2$ may be Aib, $X_{30}$ may be lysine, and $X_{34}$ may be cysteine; or
(7) $X_1$ may be histidine, $X_2$ may be Aib, $X_{30}$ may be lysine, and $X_{34}$ may be cysteine, but the GLP-2 derivative is not limited thereto

These variations for the preparation of agonists, fragments, variants, and derivatives of native GLP-2 in the present invention include all variations using L- or D-type amino acids and/or non-native amino acids; and/or variations by modification or post-translational modification (for example, methylation, acylation, ubiquitination, and intramolecular covalent bond) of the native sequence.

In the present invention, in order to protect against protein cleaving enzymes *in vivo* and increase stability, the N-terminus, C-terminus and/or the like of the GLP-2 or GLP-2 derivative may be chemically modified or protected with an organic group or the GLP-2 or GLP-2 derivative may be varied by adding an amino acid to the terminal and the like.

In particular, in the case of chemically synthesized peptides, since the N- and C-termini are charged, the N-terminus may be acetylated and/or the C-terminus may be amidated in order to remove these charges, but the variation is not particularly limited thereto.

Specifically, in the present invention, GLP-2 or a GLP-2 derivative may have its C-terminus unvaried or amidated, but is not limited thereto.

In the present invention, the insulinotropic peptide and/or GLP-2 may be in the form of a long-acting conjugate to which a biocompatible substance capable of increasing the *in vivo* half-life of each of the insulinotropic peptide and/or GLP-2 is bound, but is not limited thereto. The long-acting conjugate may exhibit enhanced persistence of efficacy compared to a derivative of insulinotropic peptide or GLP-2 to which a biocompatible substance (for example, immunoglobulin Fc region) is not bound. In the present invention, a conjugate containing an insulinotropic peptide or GLP-2 with an increased half-life by binding of a biocompatible substance to the insulinotropic peptide or GLP-2 is referred to as an "insulinotropic peptide long-acting conjugate or long-acting conjugate of insulinotropic peptide" or a "GLP-2 long-acting conjugate or long-acting conjugate of GLP-2". In the present invention, a long-acting conjugate is used interchangeably with a conjugate.

In an embodiment,

(i) the insulinotropic peptide is in the form of a long-acting conjugate to which a biocompatible substance capable of increasing *in vivo* half-life of the insulinotropic peptide is bound,
(ii) the GLP-2 is in the form of a long-acting conjugate to which a biocompatible substance capable of increasing *in vivo* half-life of the GLP-2 is bound; or
(iii) each of the insulinotropic peptide and GLP-2 is in the form of a long-acting conjugate to which a biocompatible substance capable of increasing *in vivo* half-life of each of the insulinotropic peptide and GLP-2 is bound.

Meanwhile, these conjugates may be those which do not occur naturally.
In addition, the linkage between the insulinotropic peptide or GLP-2 and the biocompatible substance (for example, immunoglobulin Fc region) in the long-acting conjugate may be a physical or chemical bond or a non-covalent or covalent bond, and may specifically be a covalent bond, but is not limited thereto.
In addition, the method of linking the insulinotropic peptide or GLP-2 and the biocompatible substance (for example, immunoglobulin Fc region) in the long-acting conjugate is not particularly limited, but the insulinotropic peptide or GLP-2 and the biocompatible substance (for example, immunoglobulin Fc region) may be linked to each other through a linker.
In addition, Korean Patent Publication No. 10-2019-0037181 regarding a long-acting conjugate of GLP-2 is incorporated herein by reference.
In a specific embodiment, the long-acting conjugate of insulinotropic peptide or GLP-2 of the present invention may have a structure represented by the following Chemical Formula 1, but is not limited thereto.

[Chem. 1] $X\text{-}L_a\text{-}F$

wherein,

X is an insulinotropic peptide or GLP-2;
L is a linker (for example, a linker containing an ethylene glycol repeating unit);
a is 0 or a natural number, provided that each L is independent of each other when a is 2 or more;
F is a biocompatible substance capable of increasing the *in vivo* half-life of X (for example, an immunoglobulin Fc region); and
the "-" is a chemical bond (for example, a covalent bond).

More specifically, X and L, and L and F may be linked to each other by a covalent bond, and in this case, the conjugate may be a conjugate in which X, L, and F are linked to each other by a covalent bond in the order in Chemical Formula 1.
In addition, F may be directly linked to X (that is, a in Chemical Formula 1 is 0) or linked through a linker (L).
In the conjugate according to the present invention, F is X, that is, a substance capable of increasing the half-life of the insulinotropic peptide or GLP-2 according to the present invention, and corresponds to a constituent of a moiety constituting the conjugate of the present invention.
The F and X may be bonded to each other by a covalent chemical bond or a non-covalent chemical bond, or F and X may be bonded to each other through L by a covalent chemical bond, a non-covalent chemical bond, or a combination thereof.
The substance capable of increasing the *in vivo* half-life of F is a biocompatible substance, and may be selected from the group consisting of, for example, a high-molecular polymer, a fatty acid, cholesterol, albumin and a fragment thereof, an albumin binding substance, a polymer of a repeating unit of a specific amino acid sequence, an antibody, an antibody fragment, an FcRn binding substance, *in vivo* connective tissue, a nucleotide, fibronectin, a transferrin, a saccharide, heparin, and elastin, but is not particularly limited thereto.
The elastin may be human tropoelastin, which is a water-soluble precursor, may be a polymer of some sequences or some repeating units of this, and includes, for example, all elastin-like polypeptides, but is not particularly limited thereto.
Examples of the high-molecular polymer may include a high-molecular polymer selected from the group consisting of polyethylene glycol (PEG), polypropylene glycol, ethylene glycol-propylene glycol copolymer, polyoxyethylated polyol, polyvinyl alcohol, polysaccharides, polyvinyl ethyl ether, biodegradable polymers, lipid polymers, chitin, hyaluronic acid, oligonucleotides, and combinations thereof, and the polysaccharide may include dextran, but is not particularly limited thereto.
In the present specification, polyethylene glycol is a term encompassing all forms of ethylene glycol homopolymer, PEG copolymer, or monomethyl-substituted PEG polymer (mPEG), but is not particularly limited thereto.
In addition, the biocompatible substance includes poly-amino acids such as poly-lysine, poly-aspartic acid, and

poly-glutamic acid, but is not limited thereto.

In addition, the fatty acid may be one having binding force with albumin *in vivo,* but is not particularly limited thereto.

As an example of the F, the F may be an FcRn-binding substance, and the FcRn-binding substance may be specifically an immunoglobulin Fc region, more specifically an IgG Fc region, still more specifically, an aglycosylated IgG4 Fc region, but the F is not particularly limited thereto.

As a specific example of the present invention, the F (for example, immunoglobulin Fc region) is a dimer composed of two polypeptide chains, and may have a structure in which one end of L is linked to only one polypeptide chain of the two polypeptide chains, but is not limited thereto.

In a specific embodiment, the long-acting conjugate of the present invention may be one in which an insulinotropic peptide or GLP-2 is linked to an immunoglobulin Fc region, but is not limited thereto.

In the present invention, the "immunoglobulin Fc region" means a heavy chain constant region excluding the heavy chain and light chain variable regions of immunoglobulin. Specifically, the immunoglobulin Fc region may include a heavy chain constant region 2 (CH2) and/or a heavy chain constant region 3 (CH3) portion, and more specifically may further include a hinge area (meaning the whole or part of the hinge area). The immunoglobulin Fc region may be a constituent constituting a moiety of the conjugate of the present invention, and specifically corresponds to F in Chemical Formula 1.

In the present specification, the Fc region includes not only the native sequence obtained from papain digestion of immunoglobulin, but also its derivatives, substitution products, and variation products, for example, a sequence in which one or more amino acid residues in the native sequence are altered by deletion, insertion, non-conservative or conservative substitution, or a combination thereof to be different from the native sequence. The derivatives, substitution products, and variation products are premised on having the ability to bind to FcRn.

The F (for example, immunoglobulin Fc region) has a structure in which two polypeptide chains are linked by a disulfide bond, and may have a structure in which the linkage is formed only through a nitrogen atom of one chain between the two chains, but is not limited thereto. The linkage through a nitrogen atom may be formed through reductive amination of the epsilon-amino atom of lysine or the N-terminal amino group.

The reductive amination reaction means a reaction in which an amine group or an amino group of a reactant reacts with an aldehyde (namely, a functional group capable of undergoing reductive amination) of another reactant to produce an amine and then an amine bond is formed by a reduction reaction, and is an organic synthesis reaction widely known in the art.

As a specific example, the immunoglobulin Fc region may be linked through its N-terminal nitrogen atom.

Such an immunoglobulin Fc region may include a hinge portion in a heavy chain constant region, but is not limited thereto.

In the present invention, the immunoglobulin Fc region may include a specific hinge sequence at the N-terminus.

In the present invention, the term "hinge sequence" refers to a site located in a heavy chain to form a dimer of immunoglobulin Fc region through an inter disulfide bond.

In the present invention, the hinge sequence may be mutated to have only one cysteine residue by deletion of part of the hinge sequence having the following amino acid sequence, but is not limited thereto:

Glu-Ser-Lys-Tyr-Gly-Pro-Pro-Cys-Pro-Ser-Cys-Pro (SEQ ID NO: 11).

The hinge sequence may include only one cysteine residue by deletion of the 8th or 11th cysteine residue in the hinge sequence of SEQ ID NO: 11. The hinge sequence of the present invention may consist of 3 to 12 amino acids including only one cysteine residue, but is not limited thereto. More specifically, the hinge sequence of the present invention may have the following sequences:

Glu-Ser-Lys-Tyr-Gly-Pro-Pro-Pro-Ser-Cys-Pro (SEQ ID NO: 12), Glu-Ser-Lys-Tyr-Gly-Pro-Pro-Cys-Pro-Ser-Pro (SEQ ID NO: 13), Glu-Ser-Lys-Tyr-Gly-Pro-Pro-Cys-Pro-Ser (SEQ ID NO: 14), Glu-Ser-Lys-Tyr-Gly-Pro-Pro-Cys-Pro-Pro (SEQ ID NO: 15), Lys-Tyr-Gly-Pro-Pro-Cys-Pro-Ser (SEQ ID NO: 16), Glu-Ser-Lys-Tyr-Gly-Pro-Pro-Cys (SEQ ID NO: 17), Glu-Lys-Tyr-Gly-Pro-Pro-Cys (SEQ ID NO: 18), Glu-Ser-Pro-Ser-Cys-Pro (SEQ ID NO: 19), Glu-Pro-Ser-Cys-Pro (SEQ ID NO: 20) , Pro-Ser-Cys-Pro (SEQ ID NO: 21), Glu-Ser-Lys-Tyr-Gly-Pro-Pro-Ser-Cys-Pro (SEQ ID NO: 22), Lys-Tyr-Gly-Pro-Pro-Pro-Ser-Cys-Pro (SEQ ID NO: 23), Glu-Ser-Lys-Tyr-Gly-Pro-Ser-Cys-Pro (SEQ ID NO: 24), Glu-Ser-Lys-Tyr-Gly-Pro-Pro-Cys (SEQ ID NO: 25), Lys-Tyr-Gly-Pro-Pro-Cys-Pro (SEQ ID NO: 26), Glu-Ser-Lys-Pro-Ser-Cys-Pro (SEQ ID NO: 27), Glu-Ser-Pro-Ser-Cys-Pro (SEQ ID NO: 28), Glu-Pro-Ser-Cys (SEQ ID NO: 29), and Ser - Cys-Pro (SEQ ID NO: 30).

More specifically, the hinge sequence may include the amino acid sequence of SEQ ID NO: 21 (Pro-Ser-Cys-Pro) or SEQ ID NO: 30 (Ser-Cys-Pro), but is not limited thereto.

In a more specific form of the long-acting conjugate of the present invention, the N-terminus of the immunoglobulin Fc region in the conjugate is proline, and the Fc region in this conjugate is linked to a linker through a nitrogen atom of the proline.

In a specific embodiment, the immunoglobulin Fc region may be in a dimeric form in which two chains of the

immunoglobulin Fc region form a homodimer or a heterodimer due to the presence of a hinge sequence. The conjugate represented by Chemical Formula 1 of the present invention may be in a form in which one end of a linker is linked to one chain of the immunoglobulin Fc region of the dimer, but is not limited thereto.

In the present invention, the term "N-terminus" refers to the amino terminus of a protein or polypeptide, and may include the most end of the amino terminus, or up to 1, 2, 3, 4, 5, 6, 7, 8, 9, or 10 or more amino acids from the most end. The immunoglobulin Fc region of the present invention may include a hinge sequence at the N-terminus, but is not limited thereto.

In addition, the immunoglobulin Fc region of the present invention may be an extended Fc region including part or whole of heavy chain constant region 1 (CH1) and/or light chain constant region 1 (CL1), excluding only the heavy and light chain variable regions of immunoglobulin as long as it has substantially equivalent or improved effect as that of the native type. In addition, the immunoglobulin Fc region may be a region in which part of a fairly long amino acid sequence corresponding to CH2 and/or CH3 is removed.

For example, the immunoglobulin Fc region of the present invention may be 1) CH1 domain, CH2 domain, CH3 domain and CH4 domain, 2) CH1 domain and CH2 domain, 3) CH1 domain and CH3 domain, 4) CH2 domain and CH3 domain, 5) a combination of one or two or more domains among CH1 domain, CH2 domain, CH3 domain, and CH4 domain with an immunoglobulin hinge region (or part of the hinge region), or 6) a dimer of each domain of a heavy chain constant region and a light chain constant region, but is not limited thereto.

In addition, as an embodiment of the long-acting conjugate of the present invention, the immunoglobulin Fc region may be in a dimeric form, one molecule of X may be covalently linked to one Fc region in a dimeric form, and in this case, the immunoglobulin Fc and X may be linked to each other through one and the same linker. Meanwhile, it is also possible for two X molecules to bind symmetrically to one Fc region in a dimeric form. In this case, the immunoglobulin Fc and X may be linked to each other through a linker. However, the immunoglobulin Fc region is not limited to the examples described above.

In addition, the immunoglobulin Fc region of the present invention includes a native amino acid sequence as well as a sequence derivative thereof. An amino acid sequence derivative means that one or more amino acid residues in a native amino acid sequence have a different sequence by deletion, insertion, non-conservative or conservative substitution, or a combination thereof.

For example, in the case of IgG Fc, amino acid residues from 214 to 238, from 297 to 299, from 318 to 322, or from 327 to 331 known to be important for binding may be used as suitable sites for variation.

In addition, a site capable of forming a disulfide bond may be removed, several amino acids at the N-terminus of native Fc may be removed, or a methionine residue may be added to the N-terminus of native Fc to obtain various kinds of derivatives. In addition, in order to eliminate the effector function, a complement binding site, for example, a C1q binding site, may be removed, and an antibody dependent cell mediated cytotoxicity (ADCC) site may be removed. Techniques for preparing such sequence derivatives of immunoglobulin Fc region are disclosed in International Patent Application Publication No. WO 97/34631, International Patent Application Publication No. 96/32478, and the like.

Amino acid exchanges in proteins and peptides that do not entirely alter the activity of the molecule are known in the art (H. Neurath, R.L. Hill, The Proteins, Academic Press, New York, 1979). The most commonly occurring exchange is between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, modification may be achieved by phosphorylation, sulfation, acrylation, glycosylation, methylation, farnesylation, acetylation, and amidation.

The above-described Fc derivative may exhibit biological activity equivalent to that of the Fc region of the present invention and may have enhanced structural stability of the Fc region against heat and pH.

In addition, such an Fc region may be obtained from a native type isolated *in vivo* from animals such as humans, cows, goats, pigs, mice, rabbits, hamsters, rats, or guinea pigs, or may be a recombinant obtained from a transformed animal cell or microorganism or its derivative. Here, the method of obtaining an Fc region from a native type may be a method of obtaining the Fc region by isolating the whole immunoglobulin from a living body of a human or animal and then treating the immunoglobulin with a proteolytic enzyme. The immunoglobulin is cleaved into Fab and Fc when treated with papain, and the immunoglobulin is cleaved into pF'c and $F(ab)_2$ when treated with pepsin. Fc or pF'c may be isolated from this by size-exclusion chromatography or the like. In a more specific embodiment, the Fc region is a recombinant immunoglobulin Fc region in which a human-derived Fc region is obtained from a microorganism.

In addition, the immunoglobulin Fc region may be a native type sugar chain, an increased sugar chain compared to the native type, a decreased sugar chain compared to the natural type, or a form in which the sugar chain is removed. Conventional methods such as chemical methods, enzymatic methods, and genetic engineering methods using microorganisms may be used to increase, decrease, or remove immunoglobulin Fc sugar chains. Here, the immunoglobulin Fc region in which the sugar chain is removed from Fc has significantly reduced ability to bind to complement (c1q), has decreased or eliminated antibody dependent cytotoxicity or complement dependent cytotoxicity, and thus does not induce unnecessary immune responses *in vivo*. In this regard, a form more suitable for its original purpose as a drug carrier is a deglycosylated or aglycosylated immunoglobulin Fc region.

In the present invention, "deglycosylation" refers to an Fc region in which sugar is removed by an enzyme, and "aglycosylation" means an Fc region that is produced by a prokaryotic animal, in a more specific embodiment, *E. coli,* and is not glycosylated.

Meanwhile, the immunoglobulin Fc region may be of human origin or animal origin such as cow, goat, pig, mouse, rabbit, hamster, rat, or guinea pig origin, and is of human origin in a more specific embodiment.

In addition, the immunoglobulin Fc region may be an Fc region derived from IgG, IgA, IgD, IgE, or IgM, a combination thereof, or a hybrid thereof. The immunoglobulin Fc region is derived from IgG or IgM, which is most abundant in human blood, in a more specific embodiment and derived from an IgG known to increase the half-life of ligand binding proteins in a still more specific embodiment. The immunoglobulin Fc region is an IgG4 Fc region in a yet still more specific embodiment, and is an aglycosylated Fc region derived from human IgG4 in the most specific embodiment, but is not limited thereto.

As an embodiment, the immunoglobulin Fc region is a fragment of human IgG4 Fc, and may be a homodimer in which two monomers are linked by a disulfide bond (inter-chain form) between cysteines, which are amino acid 3 of the respective monomers, and in this case, the homodimer has/may have a disulfide bond between cysteines 35 and 95 and a disulfide bond between cysteines 141 and 199, that is, two disulfide bonds (intra-chain form) in each monomer.

Each monomer may consist of 221 amino acids, and amino acids forming a homodimer may be a total of 442 amino acids, but are not limited thereto. Specifically, the immunoglobulin Fc region may be a homodimer including the amino acid sequence of SEQ ID NO: 35 (consisting of 442 amino acids) (here, two monomers having the amino acid sequence of SEQ ID NO: 31 (consisting of 221 amino acids) may form a homodimer by a disulfide bond between cysteines, which are amino acid 3 of the respective monomers, and the monomers of the homodimer may each independently form an internal disulfide bond between cysteines 35 and 95 and an internal disulfide bond between cysteines 141 and 199), but is not limited thereto.

Meanwhile, in the present invention, "combination" related to the immunoglobulin Fc region means that a polypeptide encoding a single-chain immunoglobulin Fc region of the same origin forms a bond with a single-chain polypeptide of a different origin when a dimer or a multimer is formed. In other words, a dimer or a multimer can be prepared from two or more Fc regions selected from the group consisting of IgG Fc, IgA Fc, IgM Fc, IgD Fc, and IgE Fc regions.

In the present invention, "hybrid" is a term meaning that sequences corresponding to immunoglobulin Fc regions of two or more different origins exist in a single-chain immunoglobulin constant region. In the case of the present invention, various kinds of hybrids are possible. In other words, hybridization of domains consisting of 1 to 4 domains from the group consisting of CH1, CH2, CH3 and CH4 of IgG Fc, IgM Fc, IgA Fc, IgE Fc, and IgD Fc is possible, and may include a hinge.

Meanwhile, IgG can also be divided into subclasses of IgG1, IgG2, IgG3 and IgG4, and combinations thereof or hybridizations thereof are also possible in the present invention. Specifically, the immunoglobulin Fc region is an IgG2 and IgG4 subclasses, and is most specifically an Fc region of IgG4 that has almost no effector function such as complement dependent cytotoxicity (CDC).

The above-described conjugate may exhibit enhanced persistence of efficacy compared to that of native insulinotropic peptide or native GLP-2 or compared to that of X not modified with F, and such a conjugate includes not only the above-described forms but also forms encapsulated in biodegradable nanoparticles, but is not limited thereto.

In Chemical Formula 1, the linker L may be a peptidic linker or a non-peptidic linker (for example, a linker containing an ethylene glycol repeating unit).

When the L is a peptidic linker, the peptidic linker may contain one or more amino acids, for example, from 1 to 1000 amino acids, but is not particularly limited thereto. In the present invention, various known peptide linkers may be used in the present invention to link F and X, and examples thereof include $[GS]_x$ linker, $[GGGS]_x$ linker, and $[GGGGS]_x$ linker, where x may be a natural number of 1 or more. However, the peptidic linker is not limited to the examples.

In the present invention, the "non-peptidic linker" includes a biocompatible polymer in which two or more repeating units are bonded. The repeating units are linked to each other by an arbitrary covalent bond but not by a peptide bond. The non-peptidic linker may be a constituent constituting a moiety of the conjugate of the present invention, and corresponds to L in Chemical Formula 1.

As the non-peptidic linker that can be used in the present invention, any polymer resistant to proteolytic enzymes *in vivo* may be used without limitation. In the present invention, the non-peptidic linker may be used interchangeably with a non-peptidic polymer.

Although not particularly limited thereto, the non-peptidic linker may be selected from the group consisting of polyethylene glycol, polypropylene glycol, a copolymer of ethylene glycol and propylene glycol, polyoxyethylated polyol, polyvinyl alcohol, polysaccharides (for example, dextran), polyvinyl ethyl ether, biodegradable polymers such as polylactic acid (PLA) and polylactic-glycolic acid (PLGA), lipid polymers, chitin, hyaluronic acid, oligonucleotides, and combinations thereof.

As the non-peptidic linker that can be used in the present invention, any polymer that is resistant to proteolytic

enzymes *in vivo* may be used without limitation, but specifically, the molecular weight of the non-peptidic polymer is in a range of more than 0 kDa to about 100 kDa, about 1 kDa to about 100 kDa, specifically in a range of about 1 kDa to about 50 kDa, about 1 kDa to about 20 kDa, about 1 kDa to about 10 kDa, or about 3.4 kDa to 10 kDa, but is not limited thereto.

Although not particularly limited thereto, the non-peptidic linker may be a linker containing an ethylene glycol repeating unit, for example, polyethylene glycol, and derivatives thereof already known in the art and derivatives that can be easily prepared at the level of skill in the art are also included in the scope of the present invention.

The repeating unit of the non-peptidic linker may be an ethylene glycol repeating unit, specifically, the non-peptidic linker may contain an ethylene glycol repeating unit and contain a functional group used in the preparation of conjugate at its terminal. The long-acting conjugate according to the present invention may be in a form in which X and F are linked through the functional group, but is not limited thereto. In the present invention, the non-peptidic linker may contain two or three or more functional groups, and the respective functional groups may be the same as or different from each other, but are not limited thereto.

Specifically, the linker may be polyethylene glycol (PEG) represented by the following Chemical Formula 4, but is not limited thereto:

[Chem. 4]

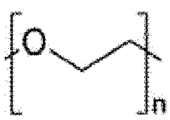

where, n = 10 to 2400, n = 10 to 480, or n = 50 to 250, but is not limited thereto.

In the long-acting conjugate, the PEG moiety may contain not only a - $(CH_2CH_2O)_n$- structure but also an oxygen atom intervening between the linking element and -$(CH_2CH_2O)_n$-, but is not limited thereto.

In a specific embodiment, the conjugate may be a structure in which an insulinotropic peptide or GLP-2 and an immunoglobulin Fc region (F) are covalently linked through a linker containing an ethylene glycol repeating unit, but is not limited thereto.

The polyethylene glycol is a term encompassing all forms of ethylene glycol homopolymer, PEG copolymer, and monomethyl-substituted PEG polymer (mPEG), but is not particularly limited thereto.

As an embodiment, the ethylene glycol repeating unit may be represented by, for example, $[OCH_2CH_2]_n$, where the n value is a natural number and may be determined such that the average molecular weight of the $[OCH_2CH_2]_n$ moiety in the peptide conjugate, for example, the number average molecular weight, is more than 0 kDa to about 100 kDa, but is not limited thereto. As another example, the n value is a natural number and the average molecular weight of the $[OCH_2CH_2]_n$ moiety in the peptide conjugate, for example, the number average molecular weight, may be about 1 kDa to about 100 kDa, about 1 kDa to about 80 kDa, about 1 kDa to about 50 kDa, about 1 kDa to about 30 kDa, about 1 kDa to about 25 kDa, about 1 kDa to about 20 kDa, about 1 kDa to about 15 kDa, about 1 kDa to about 13 kDa, about

1 kDa to about 11 kDa, about 1 kDa to about 10 kDa, about 1 kDa to about 8 kDa, about 1 kDa to about 5 kDa, about 1 kDa to about 3.4 kDa, about 3 kDa to about 30 kDa, about 3 kDa to about 27 kDa, about 3 kDa to about 25 kDa, about 3 kDa to about 22 kDa, about 3 kDa to about 20 kDa, about 3 kDa to about 18 kDa, about 3 kDa to about 16 kDa, about 3 kDa to about 15 kDa, about 3 kDa to about 13 kDa, about 3 kDa to about 11 kDa, about 3 kDa to about 10 kDa, about 3 kDa to about 8 kDa, about 3 kDa to about 5 kDa, about 3 kDa to about 3.4 kDa, about 8 kDa to about 30 kDa, about 8 kDa to about 27 kDa, about 8 kDa to about 25 kDa, about 8 kDa to about 22 kDa, about 8 kDa to about 20 kDa, about 8 kDa to about 18 kDa, about 8 kDa to about 16 kDa, about 8 kDa to about 15 kDa, about 8 kDa to about 13 kDa, about 8 kDa to about 11 kDa, about 8 kDa to about 10 kDa, about 9 kDa to about 15 kDa, about 9 kDa to about 14 kDa, about 9 kDa to about 13 kDa, about 9 kDa to about 12 kDa, about 9 kDa to about 11 kDa, about 9.5 kDa to about 10.5 kDa, about 3.4 kDa, or about 10 kDa, but is not limited thereto.

In the present invention, the term "about" is a range including all of $\pm0.5$, $\pm0.4$, $\pm0.3$, $\pm0.2$, $\pm0.1$, and the like, and includes all numerical values equivalent to or similar to the numerical values following the term "about", but is not limited thereto.

As the non-peptidic linker of the present invention bound to the immunoglobulin Fc region, not only one kind of polymer but also a combination of different kinds of polymers may be used.

In a specific embodiment, both ends of the non-peptidic linker may bind to a thiol group, an amino group, or a hydroxyl group of an immunoglobulin Fc region and a thiol group, an amino group, an azide group, or a hydroxyl group of GLP-2, but are not limited thereto.

Specifically, the non-peptidic linker may contain reactive groups capable of binding to immunoglobulin Fc and an insulinotropic peptide or GLP-2 at both ends, specifically reactive groups capable of binding to a thiol group of cysteine;

an amino group located at the N-terminus, lysine, arginine, glutamine and/or histidine; and/or a hydroxyl group located at the C-terminus in the immunoglobulin Fc region and capable of binding to a thiol group of cysteine; an amino group of lysine, arginine, glutamine and/or histidine; an azide group of azidolysine; and/or a hydroxyl group in GLP-2, but is not limited thereto.

More specifically, the reactive group of the non-peptidic polymer may be one or more selected from the group consisting of an aldehyde group, a maleimide group, and a succinimide derivative, but is not limited thereto.

In the above, examples of the aldehyde group include a propionaldehyde group or a butyl aldehyde group, but are not limited thereto.

In the above, as the succinimide derivative, succinimidyl carboxymethyl, succinimidyl valerate, succinimidyl methylbutanoate, succinimidyl methylpropionate, succinimidyl butanoate, succinimidyl propionate, *N*-hydroxysuccinimide, hydroxy succinimidyl or succinimidyl carbonate may be used, but the succinimide derivative is not limited thereto.

The non-peptidic linker may be converted into a non-peptidic polymer linkage by linking a biocompatible substance (for example, immunoglobulin Fc) with an insulinotropic peptide or GLP-2 through a reactive group as described above.

The end products produced through reductive alkylation by an aldehyde bond are much more stable than those linked by an amide bond. The aldehyde reactive group reacts selectively to the N-terminus at a low pH and can form a covalent bond with a lysine residue at a high pH, for example, under a condition of pH 9.0.

Terminal reactive groups of the non-peptidic linker of the present invention may be the same as or different from each other. The non-peptidic linker may have an aldehyde group reactive group at the ends, and the non-peptidic linker may have an aldehyde group and a maleimide reactive group at each end, or may have an aldehyde group and a succinimide reactive group at each end, but is not limited thereto.

For example, the non-peptidic linker may have a maleimide group at one end and an aldehyde group, propionaldehyde group, or butyl aldehyde group at the other end. As another example, the non-peptidic linker may have a succinimidyl group at one end and a propionaldehyde group or a butyl aldehyde group at the other end.

In a case where polyethylene glycol having a hydroxyl group at the propionic end is used as a non-peptidic linker, the conjugate of the present invention can be prepared by activating the hydroxyl group into the various reactive groups by known chemical reactions or using commercially available polyethylene glycol having a varied reactive group.

In a specific embodiment, the reactive group of the non-peptidic linker may be linked to the cysteine residue, more specifically the -SH group of cysteine in an insulinotropic peptide or GLP-2, but is not limited thereto.

If maleimide-PEG-aldehyde is used, the maleimide group may be linked to the -SH group in the insulinotropic peptide or GLP-2 by a thioether bond, and the aldehyde group may be linked to the $-NH_2$ group in immunoglobulin Fc through a reductive alkylation reaction, but the non-peptidic linker is not limited thereto, and this corresponds to one example.

Through such reductive alkylation, the N-terminal amino group in the immunoglobulin Fc region may be linked to an oxygen atom located at one end of PEG through a linker functional group having a structure of $-CH_2CH_2CH_2-$ to form a structure such as $-PEG-O-CH_2CH_2CH_2NH-$immunoglobulin Fc, and a structure in which one end of PEG is linked to a sulfur atom located in GLP-2 or at cysteine of

GLP-2 may be formed by a thioether bond. The above-mentioned thioether bond may include a structure of

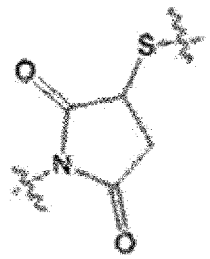

.

However, the non-peptidic linker is not particularly limited to the examples described above, and this corresponds to one example.

In the conjugate, the reactive group of the non-peptidic linker may be linked to $-NH_2$ located at the N-terminus of the immunoglobulin Fc region, but this corresponds to one example.

In the conjugate, an insulinotropic peptide or GLP-2 may be linked to a non-peptidic linker having a reactive group through the C-terminus, but this corresponds to one example.

In the present invention, "C-terminus" means the carboxy-terminus of a peptide, and refers to a position capable of binding to a non-peptidic polymer for the purpose of the present invention. For example, the C-terminus may include, but is not limited to, the most end amino acid residue of the C-terminus as well as amino acid residues around the C-terminus, specifically may include the first to twentieth amino acid residues from the most end.

As an embodiment, the conjugate represented by Chemical Formula 1 may have a structure represented by the following Chemical Formula 2 or 3.

[Chem. 2]

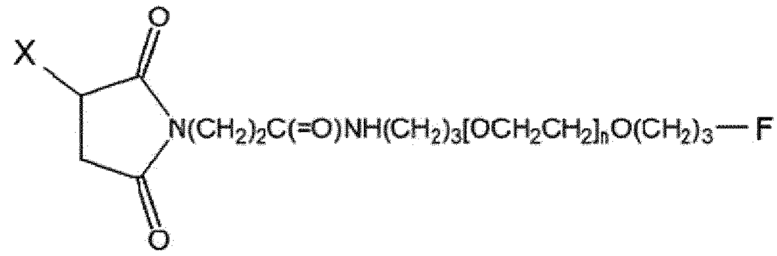

[Chem. 3] $X\text{-}(CH2)_3[OCH_2CH2]_nO(CH_2)_3\text{-}F$

In Chemical Formula 2 or 3, X may be the above-described peptide in Chemical Formula 1;

F may be an immunoglobulin Fc fragment; and
n may be a natural number. At this time, the description of n is as described above.

As an embodiment, the long-acting conjugate represented by Chemical Formula 2 may have a structure in which X of an insulinotropic peptide or GLP-2 and F of an immunoglobulin Fc region are covalently linked via an ethylene glycol repeating unit, and X may be linked to a succinimide ring in Chemical Formula 2 and F to an oxypropylene group in Chemical Formula 2, respectively. The long-acting conjugate represented by Chemical Formula 3 may have a structure in which X of an insulinotropic peptide or GLP-2 and F of an immunoglobulin Fc region are covalently linked via an ethylene glycol repeating unit, and X may be linked to an oxypropylene group in Chemical Formula 3 and F to another oxypropylene group in Chemical Formula 3, respectively.

In Chemical Formula 2 or 3, the value of n may be determined such that the average molecular weight of the $[OCH_2CH_2]_n$ moiety in the peptide conjugate, for example, the number average molecular weight is 1 kDa to 100 kDa, or 1 kDa to 20 kDa, or 10 kDa, but is not limited thereto.

In an embodiment, the succinimide ring in Chemical Formula 2 or the site where X is linked to the succinimide ring in Chemical Formula 2 may be the sulfur atom of the C-terminal cysteine of X. The oxypropylene group in Chemical Formula 3 or the site where X is linked to the oxypropylene group in Chemical Formula 3 may be the sulfur atom of the C-terminal cysteine of X.

The site in F linked to the oxypropylene group in Chemical Formula 2 or Chemical Formula 3 is not particularly limited. In an embodiment of the present invention, the site of F linked to the oxypropylene group may be N-terminal nitrogen or the nitrogen atom of a residue inside F (for example, epsilon nitrogen of lysine). In a specific embodiment of the present invention, the site where F is linked to the oxypropylene group in Chemical Formula 2 or Chemical Formula 3 may be the N-terminal proline of F, but is not limited thereto.

Meanwhile, the insulinotropic peptide, GLP-2, or a long-acting conjugate thereof according to the present invention includes all forms of itself, a salt thereof (for example, a pharmaceutically acceptable salt), or a solvate thereof.

The insulinotropic peptide, GLP-2, or a long-acting conjugate thereof may be in any pharmaceutically acceptable form.

The kind of salt is not particularly limited. However, the salt is preferably in a form that is safe and effective for a subject, for example, a mammal, but is not particularly limited thereto.

In the present invention, the term "pharmaceutically acceptable salt" includes salts derived from pharmaceutically acceptable inorganic acids, organic acids, or bases. Examples of suitable acids include hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-*p*-sulfonic acid, tartaric acid, acetic acid, citric acid, methanesulfonic acid, formic acid, benzoic acid, malonic acid, naphthalene-2-sulfonic acid, and benzenesulfonic acid. Salts derived from suitable bases may contain alkali metals such as sodium and potassium, alkaline earth metals such as magnesium, and ammonium.

In the present invention, the term "solvate" refers to a complex formed from a peptide, compound, or salt thereof according to the present invention and a solvent molecule.

The native insulinotropic peptide, varied insulinotropic peptide, and GLP-2 of the present invention may be

synthesized through a solid phase synthesis method, can also be produced by a recombinant method, and may be prepared by requesting commercially.

As another embodiment, the combination, pharmaceutical composition, or pharmaceutical kit of the present invention may contain

(i) an insulinotropic peptide and GLP-2,
(ii) an insulinotropic peptide long-acting conjugate bound to a biocompatible substance capable of increasing its *in vivo* half-life; and GLP-2,
(iii) an insulinotropic peptide; and a GLP-2 long-acting conjugate bound to a biocompatible substance capable of increasing its *in vivo* half-life, or
(iv) an insulinotropic peptide long-acting conjugate bound to a biocompatible substance capable of increasing its *in vivo* half-life; and a GLP-2 long-acting conjugate bound to a biocompatible substance capable of increasing its *in vivo* half-life.

The combination, pharmaceutical composition or pharmaceutical kit of the present invention can be used to prevent, improve, or treat short bowel syndrome.

In the present invention, the term "prevention" refers to any action that inhibits or delays the onset of a target disease, for example, short bowel syndrome by administration of a combination or composition containing an insulinotropic peptide and GLP-2, and the term "treatment" refers to any action that improves or benefits the symptoms of a target disease, for example, short bowel syndrome by administration of a combination or composition containing an insulinotropic peptide and GLP-2. The term "improvement" refers to any action that at least diminishes a parameter, for example, the severity of symptom associated with the condition being treated by administration of a combination or composition of the present invention.

In the present invention, the term "administration" means introducing a predetermined substance into a patient by any proper method, and the route of administration of the composition is not particularly limited thereto, but may be any general route through which the composition can reach the target *in vivo,* and the administration may be, for example, intraperitoneal administration, intravenous administration, intramuscular administration, subcutaneous administration, intradermal administration, oral administration, topical administration, intranasal administration, intrapulmonary administration, and intrarectal administration.

The insulinotropic peptide and GLP-2 may be used in combination to prevent or treat short bowel syndrome.

In the present invention, "short bowel syndrome (SBS)" is a digestive malabsorption disorder caused by congenital shortness of the small intestine or acquired loss (reduced absorption area of the small intestine) of a certain percentage (for example, 50% or more) of the small intestine due to surgery and the like, and the symptoms may include lack of nutrient absorption, malabsorption of nutrients, abdominal pain, diarrhea, steatorrhea, lactose intolerance, dehydration, weight loss, lethargy, fatigue, and the like.

When administered to a subject, the combination or composition according to the present invention can cause one or more among weight gain, an increase in small intestine length, a decrease in gastrointestinal motility, and an increase in nutrient absorption to prevent, improve, or treat short bowel syndrome, but is not limited thereto.

The pharmaceutical composition of the present invention may further contain a pharmaceutically acceptable carrier, excipient, or diluent. Such a pharmaceutically acceptable carrier, excipient, or diluent may be one that does not occur naturally.

In the present invention, the term "pharmaceutically acceptable" means an amount that is sufficient to exhibit a therapeutic effect and does not cause side effects, and may be easily determined by those skilled in the art according to factors well known in the medical field, such as kind of disease, patient's age, weight, health, and sex, patient's sensitivity to drugs, route of administration, method of administration, number of administration, treatment period, and drugs compounded or used simultaneously.

The pharmaceutical composition of the present invention may further contain a pharmaceutically acceptable excipient. The excipient is not particularly limited thereto, but binders, lubricants, disintegrants, solubilizers, dispersants, stabilizers, suspending agents, pigments, flavors, and the like may be used at the time of oral administration, buffers, preservatives, soothing agents, solubilizers, isotonic agents, stabilizers, and the like may be used in mixture in the case of an injection, and bases, excipients, lubricants, preservatives, and the like may be used in the case of topical administration.

The formulation of the composition of the present invention may be variously prepared by mixing the composition with pharmaceutically acceptable excipients as described above. For example, the composition may be prepared in the form of tablets, troches, capsules, elixirs, suspensions, syrups, wafers, and the like at the time of oral administration, and the composition may be prepared in a unit dosage ampoule or multiple dosage form in the case of an injection. In addition, the composition may be formulated into solutions, suspensions, tablets, pills, capsules, sustained-release preparations, and the like.

As examples of carriers, excipients, and diluents suitable for preparations, lactose, dextrose, sucrose, sorbitol, mannitol, xylitol, erythritol, maltitol, starch, acacia, alginates, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, mineral oil or the like may be used. In addition, fillers, anti-coagulants, lubricants, wetting agents, flavoring agents, preservatives, and the like may be further contained.

The pharmaceutical composition of the present invention may have any one formulation selected from the group consisting of tablets, pills, powders, granules, capsules, suspensions, oral liquids, emulsions, syrups, sterilized aqueous solutions, non-aqueous solvents, lyophilized preparations, and suppositories.

The composition may be formulated into a preparation in a unit dosage form suitable for administration into the body of a patient, specifically, a preparation useful for the administration of a protein drug according to a conventional method in the pharmaceutical field, and administered by an oral administration route or a parenteral administration route including a cutaneous, intravenous, intramuscular, intraarterial, intramedullary, intrathecal, intraventricular, pulmonary, transdermal, subcutaneous, intraperitoneal, intranasal, intragastric, topical, sublingual, intravaginal or rectal route using an administration method commonly used in the art, but is not limited thereto.

The conjugate may be used in mixture with various pharmaceutically acceptable carriers such as physiological saline or organic solvents, and carbohydrates such as glucose, sucrose or dextran, antioxidants such as ascorbic acid or glutathione, chelating agents, low molecular weight proteins, other stabilizers, or the like may be used as agents in order to increase stability or absorption.

The dose and number of administration of the pharmaceutical composition of the present invention are determined according to the kind of drug as an active ingredient together with various related factors such as the disease to be treated, the route of administration, the patient's age, sex and weight, and the severity of the disease. Specifically, the composition of the present invention may contain the peptide or a long-acting conjugate containing the same in a pharmaceutically effective amount, but is not limited thereto.

To contain the insulinotropic peptide or GLP-2 in a pharmaceutically effective amount means the degree to which the desired pharmacological activity due to the insulinotropic peptide or GLP-2 can be acquired, and may mean a pharmaceutically acceptable level that does not cause toxicity or side effects in the administered subject or causes insignificant toxicity or side effects, but is not limited thereto. Such a pharmaceutically effective amount may be determined by comprehensively considering the number of administrations, patients, formulation, and the like.

Although not particularly limited thereto, the pharmaceutical composition of the present invention may contain the ingredient (active ingredient) in an amount of 0.01 to 99 weight by volume%.

The total effective amount of the composition of the present invention may be administered to a patient in a single dose or by a fractionated treatment protocol in which multiple doses are administered over a long period of time. The pharmaceutical composition of the present invention may contain the active ingredient at different contents according to the severity of the disease. Specifically, a preferred total dose of the peptide or conjugate of the present invention may be about 0.0001 mg to 500 mg per 1 kg of the patient's weight per day, but is not limited thereto. However, the effective dose of the conjugate for a patient is determined by considering various factors, such as the patient's age, weight, health condition, sex, diet and excretion rate and the severity of the disease, as well as the route of administration of the pharmaceutical composition and the number of treatments, and thus those skilled in the art will be able to determine an appropriate effective dose according to the specific use of the composition of the present invention considering this point. The pharmaceutical composition according to the present invention is not particularly limited in its formulation, administration route and administration method as long as it exhibits the effects of the present invention.

The pharmaceutical composition of the present invention has excellent *in vivo* persistence and potency, so that the number and frequency of administration of the pharmaceutical preparation of the present invention can be significantly decreased, but is not limited thereto.

Another aspect embodying the present invention provides a pharmaceutical composition for the prevention, improvement, or treatment of short bowel syndrome containing the insulinotropic peptide, in which the pharmaceutical composition is used in combination with GLP-2.

The insulinotropic peptide, GLP-2, prevention, treatment, improvement, use in combination, short bowel syndrome, and pharmaceutical composition are as described above.

Another aspect embodying the present invention provides a food composition for the prevention or improvement of short bowel syndrome containing an insulinotropic peptide, in which the insulinotropic peptide is administered in combination with GLP-2.

The insulinotropic peptide, GLP-2, prevention, improvement, use in combination, and short bowel syndrome are as described above.

The food composition may be used as a health functional food. In a case where the composition of the present invention is used as a food supplement additive, the insulinotropic peptide, GLP-2, a long-acting conjugate containing the same, or a combination thereof may be added as it is or used together with other foods or food ingredients, and may be appropriately used according to conventional methods. The amount of the active ingredient mixed may be appropriately

determined depending on the purpose of use (prevention, health, or therapeutic treatment).

In the present invention, the term "health functional food" refers to food manufactured and processed using specific ingredients as raw materials or by methods such as extraction, concentration, purification, and mixing of specific ingredients contained in food materials for the purpose of health supplementation, and refers to food designed and processed so as to fully exert the bioregulatory functions such as biodefense, regulation of biorhythm, prevention of disease, and recovery from disease with respect to a living body by the above ingredients, and the composition for health food can perform functions related to prevention of disease, recovery from diseases, and the like.

Another aspect embodying the present invention provides a method for preventing, improving, or treating short bowel syndrome, which includes administering an insulinotropic peptide and GLP-2 to a subject in need thereof.

As another embodiment, the method may be a method for preventing, improving, or treating short bowel syndrome, which includes administering and/or using the combination, pharmaceutical composition, or pharmaceutical kit of the present invention in a subject in need thereof.

As another embodiment, the method is a method for preventing, improving, or treating short bowel syndrome, which includes administering and/or using in combination with a composition containing an insulinotropic peptide in a pharmaceutically effective amount and a composition containing GLP-2 in a pharmaceutically effective amount in a subject in need thereof.

The insulinotropic peptide, GLP-2, prevention, treatment, improvement, short bowel syndrome, use in combination, combination, pharmaceutical composition, and pharmaceutical kit are as described above.

In the present invention, the subject is a subject suspected of short bowel syndrome, and the subject suspected of short bowel syndrome refers mammals, including mice, livestock, and the like, including humans, who have or may develop the disease, but subjects that can be treated with the composition of the present invention are included without limitation. By administering the composition of the present invention to a subject suspected of short bowel syndrome disease, the subject can be effectively treated. Short bowel syndrome is as described above.

The method of the present invention may include administering a pharmaceutically effective amount of a combination or pharmaceutical composition containing an insulinotropic peptide and GLP-2. In the method according to the present invention, an insulinotropic peptide and GLP-2 may be administered as one preparation, or separate preparations thereof may be administered simultaneously, separately, sequentially, or in reverse order, but the method is not limited thereto.

The proper total daily amount may be determined by a treating physician within the scope of sound medical judgment, and may be administered once or in a manner divided into several times. However, for the purposes of the present invention, the specific therapeutically effective amount for a particular patient is preferably applied differently depending on the kind and degree of response to be achieved and, in some cases, whether other preparations are used as well as various factors including the specific composition, patient's age, weight, general health condition, sex and diet, administration time, administration route and secretion rate of the composition, treatment period, and drugs used together with or simultaneously used with a specific composition, and similar factors well known in the field of medicine.

Another aspect embodying the present invention is the use of the combination, pharmaceutical compositions, or pharmaceutical kit for the prevention, improvement, or treatment of short bowel syndrome and/or use of the combination, pharmaceutical compositions, or pharmaceutical kit for the preparation of a medicament for the prevention or treatment of short bowel syndrome.

Hereinafter, the present invention will be described in more detail with reference to the following Examples. However, the following Examples are only for exemplifying the present invention, and the scope of the present invention is not limited thereto.

## Example 1: Preparation of GLP-1 derivative long-acting conjugate

### 1-1. Preparation of CA-exendin-4-PEG conjugate

A PEGylated peptide conjugate (mono-PEGylated CA-exendin-4) was prepared by a covalent bond between the amine group of the 27th lysine residue in CA-exendin-4 (*N*-[2-(1*H*-imidazol-5-yl)acetyl]-exendin-4, Hanmi Fine Chemical, Korea) peptide and polyethylene glycol having an aldehyde (ALD) group at both ends (3.4 kDa, ALD(2) PEG, Hanmi Fine Chemical, Korea). Specifically, the reaction was conducted at a molar ratio of CA-exendin-4 : ALD(2) PEG of 1:5 to 1:15, a concentration of CA-exendin-4 of 6 mg/mL to 12 mg/mL, and a temperature of 4°C to 10°C to room temperature for about 4 to 12 hours. At this time, the reaction was conducted in 0.1 M HEPES with a pH of 7.0 to 8.5 and about 45% isopropanol, and sodium cyanoborohydride (NaCNBHs) at a concentration of 2 mM to 50 mM was added for the reaction. The reaction mixture was purified using Source 15S (Cytiva, USA) column using a buffer containing sodium citrate (pH of 2.0 to 3.5) and about 45% ethanol and potassium chloride concentration gradient.

1-2. Preparation of CA-exendin-4-PEG-Fc conjugate

In order to prepare a CA-exendin-4-PEG-Fc conjugate, an immunoglobulin Fc fragment was added to the mono-PEGylated CA-exendin-4 obtained in Example 1-1 to have a total protein concentration of 10 mg/mL to 50 mg/mL, followed by reaction at 4°C to 10°C to room temperature for about 12 to 17 hours. At this time, the reaction solution was 0.1 M potassium phosphate with a pH of 5.5 to 8.5, and 2 mM to 50 mM sodium cyanoborohydride was added as a reducing agent. After the reaction was completed, the CA-exendin-4-PEG-Fc conjugate in the reaction mixture was purified using two kinds of columns, hydrophobic binding and anion exchange columns. The hydrophobic binding column, Source 15Phenyl (Cytiva, USA) isolated and purified the unreacted immunoglobulin Fc fragments using Tris-HCl (pH 7.5) buffer and NaCl concentration gradient, and the anion exchange column, Source 15Q (Cytiva, USA) isolated and purified the CA-exendin-4-PEG-Fc conjugate by removing overreacted impurities using Tris-HCl (pH 7.5) buffer and NaCl concentration gradient.

**Example 2: Preparation of GLP-2 derivative long-acting conjugate**

2-1. Preparation of CA GLP-2 RK-PEG conjugate

In order to PEGylate the GLP-2 derivative CA GLP-2 RK of SEQ ID NO: 4 in Table 1 with ALD (2) PEG (modified polyethylene glycol in which the hydrogen atoms at both ends were each substituted with a propionaldehyde group (3-oxopropyl group) and the chemical formula weight of the ethylene glycol repeating unit moiety was 3.4 kDa, manufactured by NOF, Japan), modified polyethylene glycol, the reaction was conducted at a molar ratio of the GLP-2 derivative to ALD(2) PEG of 1:5 to 1:20, a concentration of the GLP-2 derivative of 5 mg/mL to 10 mg/mL, and a temperature of 2°C to 8°C for 4 to 16 hours. At this time, the reaction was conducted in 20 mM HEPES with pH 7.5 and ethanol, and 20 mM sodium cyanoborohydride was added as a reducing agent for the reaction. The reaction mixture was subjected to the purification of mono-PEGylated GLP-2 derivative using Source 15S (GE, USA) column using a buffer solution containing sodium citrate with pH 2.0 and ethanol and potassium chloride concentration gradient.

2-2. Preparation of CA GLP-2 RK-PEG-Fc conjugate

Next, the reaction was conducted at a molar ratio of the purified mono-PEGylated GLP-2 derivative to the immunoglobulin Fc fragment of 1:2 to 1:6, a total protein concentration of 30 mg/mL to 35 mg/mL, and a temperature of 2°C to 8°C for 12 to 20 hours. At this time, the reaction solution was 100 mM potassium phosphate buffer (pH 6.0) and isopropanol, and 20 mM sodium cyanoborohydride was added as a reducing agent.

After the reaction was completed, the reaction mixture was applied to Source 15Q (GE, USA) column using a bis-Tris pH 6.5 buffer and sodium chloride concentration gradient, and applied to Source 15 ISO (GE, USA) using the concentration gradient of ammonium sulfate and sodium citrate with a pH of 5.0 to 5.2 to purify a long-acting conjugate of a GLP-2 derivative, which was a conjugate in which the GLP-2 derivative was covalently linked to an immunoglobulin Fc fragment through a polyethylene glycol linker.

**Example 3: Examination of increase in mouse weight and small intestine length by combined administration of GLP-1 derivative long-acting conjugate and GLP-2 derivative long-acting conjugate**

Mice were used to measure the efficacy of improving the *in vivo* weight and small intestine length by combined administration of the GLP-1 derivative long-acting conjugate and the GLP-2 derivative long-acting conjugate. Specifically, 7-week-old male mice (C57BL/6) were divided into 5 mice per group as follows:

- G1: normal control group (drug-free group, vehicle),
- G2: group administered with GLP-1 derivative long-acting conjugate (0.039 mg/kg/Q2D) (GLP-1 derivative long-acting conjugate 0.039 mg/kg/Q2D, 2 weeks),
- G3: group administered with GLP-2 derivative long-acting conjugate (0.889 mg/kg/Q2D) (GLP-2 derivative long-acting conjugate 0.889 mg/kg/Q2D, 2 weeks), and
- G4: group administered with GLP-1 derivative long-acting conjugate (0.039 mg/kg/Q2D) and GLP-2 derivative long-acting conjugate (0.889 mg/kg/Q2D) ([GLP-1 derivative long-acting conjugate 0.039 mg/kg/Q2D + GLP-2 derivative long-acting conjugate 0.889 mg/kg/Q2D], 2 weeks).

In the present specification, the numerical value expressed as the dose of the GLP-1 derivative long-acting conjugate or the GLP-2 derivative long-acting conjugate is a numerical value excluding the mass of the polyethylene glycol linker moiety from the total mass of the GLP-1 derivative long-acting conjugate or GLP-2 derivative long-acting

conjugate used, that is, a numerical value based on the sum of the masses of only the polypeptide moieties.

The mouse weight was observed while repeated administration was performed for 2 weeks (D0-D14: the first day of drug administration was designated as day 0). Then, on D14, the length of the small intestine was measured through autopsy.

As a result, it has been confirmed that weight loss due to GLP-1 is not observed in the group administered with the GLP-1 derivative long-acting conjugate and the GLP-2 derivative long-acting conjugate in combination compared to the group administered with the GLP-1 derivative long-acting conjugate singly (FIG. 1), and the length of the small intestine is significantly increased in the group administered with the GLP-1 derivative long-acting conjugate and the GLP-2 derivative long-acting conjugate in combination compared to the group administered with the GLP-1 derivative long-acting conjugate singly or the group administered with the GLP-2 derivative long-acting conjugate singly (FIG. 2).

### Example 4: Decrease in gastrointestinal motility by combined administration of GLP-1 derivative long-acting conjugate and GLP-2 derivative long-acting conjugate

Mice were used to measure the effect of decreasing gastrointestinal motility *in vivo* by combined administration of the GLP-1 derivative long-acting conjugate and the GLP-2 derivative long-acting conjugate. Specifically, 7-week-old male mice (C57BL/6) were divided into 6 mice per group as follows:

- G1: normal control group (drug-free group, vehicle),
- G2: group administered with GLP-1 derivative long-acting conjugate (0.136 mg/kg) (GLP-1 derivative long-acting conjugate 0.136 mg/kg),
- G3: group administered with GLP-2 derivative long-acting conjugate (3.111 mg/kg) (GLP-2 derivative long-acting conjugate 3.111 mg/kg), and
- G4: group administered with GLP-1 derivative long-acting conjugate (0.136 mg/kg) and GLP-2 derivative long-acting conjugate (3.111 mg/kg) (GLP-1 derivative long-acting conjugate 0.136 mg/kg + GLP-2 derivative long-acting conjugate 3.111 mg/kg).

Thereafter, the drug was administered once with fasting. After 24 hours, 5% charcoal meal (w/v in 10% Gum Arabic, 0.3 mL per mouse) was administered orally. After 20 minutes, the small intestine was taken through autopsy, the movement distance of the charcoal meal and the total length of the small intestine were measured, and the small intestine transit of charcoal meal was calculated using the following equation.

$$\text{Small intestine transit of charcoal meal (\%)} \\ = (\text{charcoal meal movement distance/total small intestine length}) \times 100$$

These results suggest that the combined administration of a GLP-1 derivative long-acting conjugate and a GLP-2 derivative long-acting conjugate of the present invention can be effective in treating short bowel syndrome as the combined administration rapidly increases the length of the small intestine without causing weight loss due to GLP-1 while also leading to a GLP-2-induced increase in the length of the small intestine and increasing the patient's nutrient absorption through a decrease in gastrointestinal motility (FIG. 3) compared to the single administration groups.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes and modifications that fall within metes and bounds of the claims or equivalents of such metes and bounds are therefore intended to be embraced by the claims.

## Claims

1. A pharmaceutical composition for prevention or treatment of short bowel syndrome comprising an insulinotropic peptide in a pharmaceutically effective amount, wherein the pharmaceutical composition is administered in combination with glucagon-like peptide-2 (GLP-2).

2. A pharmaceutical composition for prevention or treatment of short bowel syndrome comprising GLP-2 in a pharmaceutically effective amount, wherein the pharmaceutical composition is administered in combination with an insulinotropic peptide.

3. The pharmaceutical composition according to claim 1 or 2, wherein the insulinotropic peptide is selected from the group consisting of glucagon-like peptide-1 (GLP-1); exendin-3; exendin-4; agonists, derivatives, fragments, and variants of the glucagon-like peptide-1 (GLP-1), exendin-3, and exendin-4; and combinations thereof.

4. The pharmaceutical composition according to claim 1 or 2, wherein the insulinotropic peptide is an insulinotropic peptide derivative in which a N-terminal histidine residue of an insulinotropic peptide is substituted with imidazoacetyldeshistidine, desaminohistidine, β-hydroxyimidazopropionyldeshistidine, N-dimethylhistidine, or β-carboxyimidazopropionyldeshistidine.

5. The pharmaceutical composition according to claim 1 or 2, wherein the insulinotropic peptide is native exendin-4, an exendin-4 derivative in which an *alpha*-carbon of a histidine residue, which is a first amino acid at a N-terminus of exendin-4, and a N-terminal amino group bound to the alpha-carbon are removed, an exendin-4 derivative in which a N-terminal amino group of exendin-4 is removed, an exendin-4 derivative in which a N-terminal amino group of exendin-4 is substituted with a hydroxyl group, an exendin-4 derivative in which a N-terminal amino group of exendin-4 is modified with two methyl groups, an exendin-4 derivative in which a N-terminal amino group of exendin-4 is substituted with a carboxyl group, an exendin-4 derivative in which a twelfth amino acid (lysine) of exendin-4 is substituted with serine, or an exendin-4 derivative in which a twelfth amino acid (lysine) of exendin-4 is substituted with arginine.

6. The pharmaceutical composition according to claim 1 or 2, wherein the GLP-2 is native GLP-2 or a GLP-2 derivative.

7. The pharmaceutical composition according to claim 6, wherein the GLP-2 derivative is a GLP-2 derivative in which at least one amino acid in a native GLP-2 sequence is subjected to variation selected from the group consisting of substitution, addition, deletion, modification, and a combination thereof.

8. The pharmaceutical composition according to claim 6, wherein the GLP-2 derivative is subjected to variation of at least one amino acid among amino acids 1, 2, 30, and 33 in SEQ ID NO: 1.

9. The pharmaceutical composition according to claim 6, wherein the GLP-2 derivative includes an amino acid sequence represented by the following Formula 1:

[Formula 1] $X_1X_2$DGSFSDEMNTILDNLAARDFINWLIQT$X_{30}$ITD$X_{34}$ (SEQ ID NO: 9)

wherein,

$X_1$ is histidine, imidazoacetyldeshistidine, desaminohistidine, β-hydroxyimidazopropionyldeshistidine, *N*-dimethylhistidine, or β-carboxyimidazopropionyldeshistidine;
$X_2$ is alanine, glycine, or 2-aminoisobutyric acid (Aib);
$X_{30}$ is lysine or arginine; and
$X_{34}$ is absent or is lysine, arginine, glutamine, histidine, 6-azidolysine, or cysteine;
provided that the sequence of SEQ ID NO: 1 is excluded from the amino acid sequences represented by Formula 1.

10. The pharmaceutical composition according to claim 9, wherein in the GLP-2 derivative

(1) $X_1$ is imidazoacetyldeshistidine, $X_2$ is glycine, $X_{30}$ is lysine, and $X_{34}$ is cysteine;
(2) $X_1$ is imidazoacetyldeshistidine, $X_2$ is glycine, $X_{30}$ is lysine, and $X_{34}$ is lysine;
(3) $X_1$ is imidazoacetyldeshistidine, $X_2$ is glycine, $X_{30}$ is arginine, and $X_{34}$ is lysine;
(4) $X_1$ is imidazoacetyldeshistidine, $X_2$ is glycine, $X_{30}$ is lysine, and $X_{34}$ is 6-azidolysine;
(5) $X_1$ is imidazoacetyldeshistidine, $X_2$ is glycine, $X_{30}$ is arginine, and $X_{34}$ is cysteine;
(6) $X_1$ is imidazoacetyldeshistidine, $X_2$ is Aib, $X_{30}$ is lysine, and $X_{34}$ is cysteine; or
(7) $X_1$ is histidine, $X_2$ is Aib, $X_{30}$ is lysine, and $X_{34}$ is cysteine.

11. The pharmaceutical composition according to claim 6, wherein the GLP-2 derivative includes an amino acid sequence represented by the following Formula 2:

[Formula 2] $X_1X_2$DGSFSDEMNTILDNLAARDFINWLIQT$X_{30}$ITD$X_{34}$ (SEQ ID NO: 10)

wherein,

$X_1$ is histidine, imidazoacetyldeshistidine, desaminohistidine, β-hydroxyimidazopropionyldeshistidine, *N*-dimethylhistidine, or β-carboxyimidazopropionyldeshistidine;
$X_2$ is alanine, glycine, or 2-aminoisobutyric acid (Aib);
$X_{30}$ is lysine or arginine; and
$X_{34}$ is one or more arbitrary amino acids or one or more arbitrary amino acids subjected to variation;
provided that the sequence of SEQ ID NO: 1 is excluded from the amino acid sequences represented by Formula 2.

**12.** The pharmaceutical composition according to claim 6, wherein the GLP-2 derivative is a peptide having an amino acid sequence selected from the group consisting of SEQ ID NOs: 2 to 8.

**13.** The pharmaceutical composition according to claim 1 or 2, wherein the composition, when administered to a subject, causes one or more among weight gain, an increase in small intestine length, a decrease in gastrointestinal motility, and an increase in nutrient absorption.

**14.** The pharmaceutical composition according to claim 1 or 2, wherein the insulinotropic peptide and GLP-2 have a C-terminus unvaried or amidated.

**15.** The pharmaceutical composition according to claim 1 or 2, wherein

(i) the insulinotropic peptide is in a form of a long-acting conjugate to which a biocompatible substance capable of increasing *in vivo* half-life of the insulinotropic peptide is bound,
(ii) the GLP-2 is in a form of a long-acting conjugate to which a biocompatible substance capable of increasing *in vivo* half-life of the GLP-2 is bound; or
(iii) each of the insulinotropic peptide and GLP-2 is in a form of a long-acting conjugate to which a biocompatible substance capable of increasing *in vivo* half-life of each of the insulinotropic peptide and GLP-2 is bound.

**16.** The pharmaceutical composition according to claim 15, wherein the conjugate is represented by the following Chemical Formula 1:

[Chem. 1] $X\text{-}L_a\text{-}F$

wherein,

X is an insulinotropic peptide or GLP-2;
L is a linker containing an ethylene glycol repeating unit;
a is 0 or a natural number, provided that each L is independent of each other when a is 2 or more;
F is an immunoglobulin Fc region; and
the "-" is a covalent bond.

**17.** The pharmaceutical composition according to claim 16, wherein the immunoglobulin Fc region is an aglycosylated IgG4 Fc region.

**18.** The pharmaceutical composition according to claim 16, wherein the F is a dimer composed of two polypeptide chains, and one end of L is linked only to one polypeptide chain of the two polypeptide chains.

**19.** The pharmaceutical composition according to claim 16, wherein the L is polyethylene glycol.

**20.** The pharmaceutical composition according to claim 16, wherein a chemical formula weight of an ethylene glycol repeating unit moiety in the L is in a range of 1 kDa to 100 kDa.

**21.** The pharmaceutical composition according to claim 1 or 2, further comprising a pharmaceutically acceptable carrier, excipient, or diluent.

**22.** The pharmaceutical composition according to claim 1 or 2, wherein the composition containing an insulinotropic peptide is administered in combination with the composition containing GLP-2 simultaneously, sequentially, or in reverse order.

[FIG. 1]

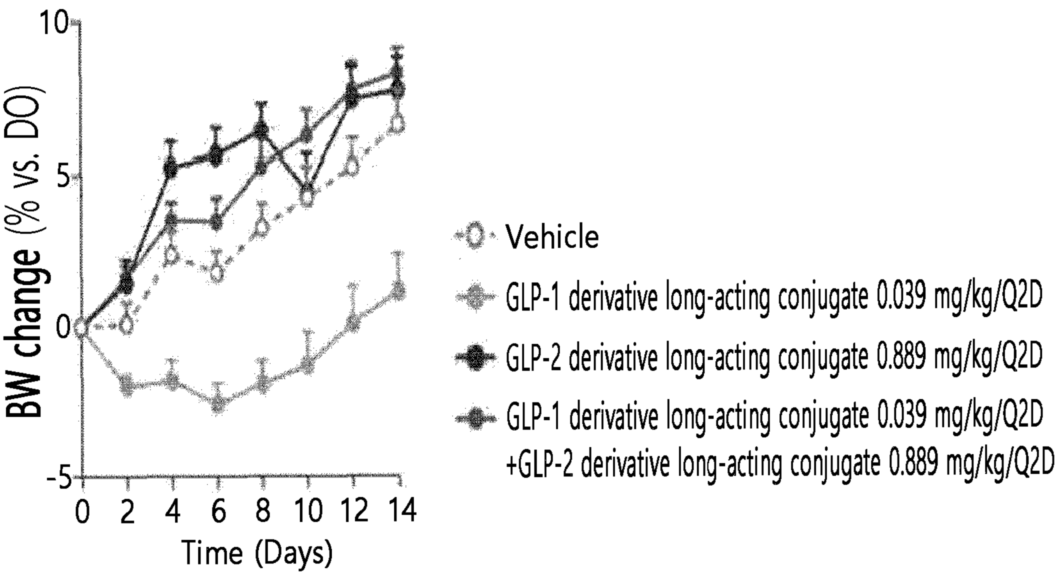
10
5
0
-5
BW change (% vs. DO)
0 2 4 6 8 10 12 14
Time (Days)
Vehicle
GLP-1 derivative long-acting conjugate 0.039 mg/kg/Q2D
GLP-2 derivative long-acting conjugate 0.889 mg/kg/Q2D
GLP-1 derivative long-acting conjugate 0.039 mg/kg/Q2D
+GLP-2 derivative long-acting conjugate 0.889 mg/kg/Q2D

[FIG. 2]

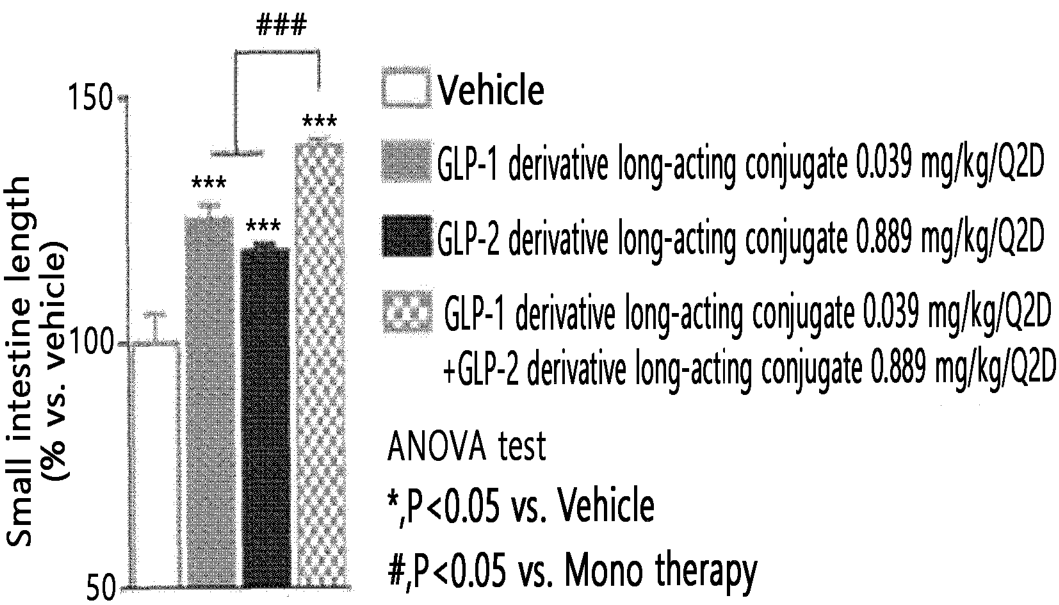
###
***
***
***
150
100
50
Small intestine length
(% vs. vehicle)
Vehicle
GLP-1 derivative long-acting conjugate 0.039 mg/kg/Q2D
GLP-2 derivative long-acting conjugate 0.889 mg/kg/Q2D
GLP-1 derivative long-acting conjugate 0.039 mg/kg/Q2D
+GLP-2 derivative long-acting conjugate 0.889 mg/kg/Q2D
ANOVA test
*,P<0.05 vs. Vehicle
#,P<0.05 vs. Mono therapy

[FIG. 3]

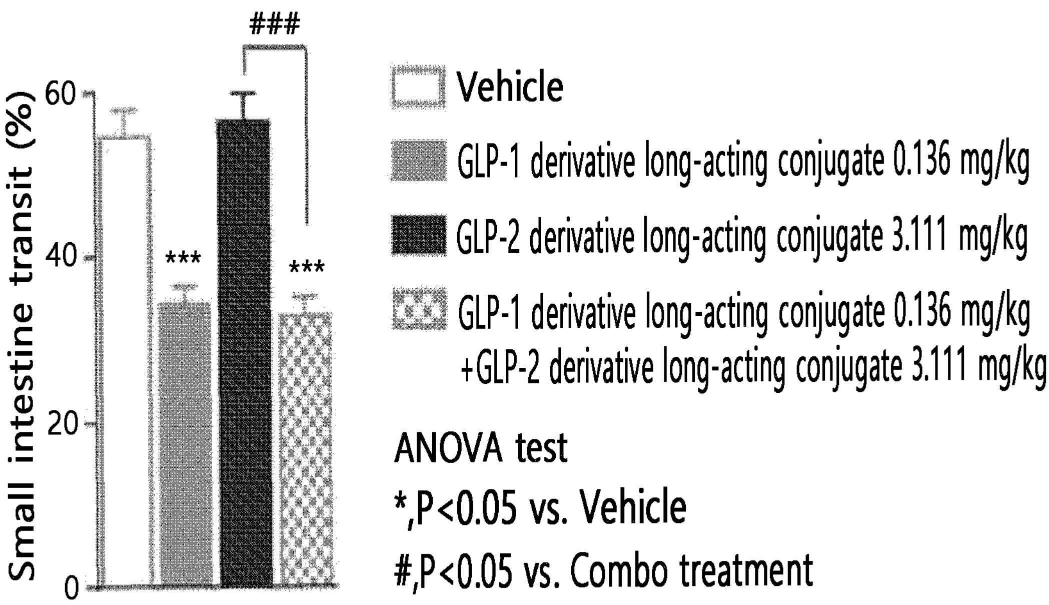
Small intestine transit (%)
60
40
20
0
###
***
***
Vehicle
GLP-1 derivative long-acting conjugate 0.136 mg/kg
GLP-2 derivative long-acting conjugate 3.111 mg/kg
GLP-1 derivative long-acting conjugate 0.136 mg/kg
+GLP-2 derivative long-acting conjugate 3.111 mg/kg
ANOVA test
*,P<0.05 vs. Vehicle
#,P<0.05 vs. Combo treatment

# INTERNATIONAL SEARCH REPORT

International application No.
**PCT/KR2021/019854**

## A. CLASSIFICATION OF SUBJECT MATTER

**A61K 38/26**(2006.01)i; **A61K 38/22**(2006.01)i; **A61K 47/68**(2017.01)i; **A61P 1/00**(2006.01)i; **A61P 29/00**(2006.01)i; **C07K 14/605**(2006.01)i

According to International Patent Classification (IPC) or to both national classification and IPC

## B. FIELDS SEARCHED

Minimum documentation searched (classification system followed by classification symbols)

A61K 38/26(2006.01); A61K 38/00(2006.01); A61P 3/10(2006.01); C07K 14/575(2006.01); C07K 14/605(2006.01); C07K 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 단장증후군(short bowel syndrome), 인슐린(insulin), 글루카곤 유사 펩타이드-1(glucagon-like peptide-1, GLP-1), 글루카곤 유사 펩타이드-2(glucagon-like peptide-2, GLP-2), 엑센딘-4(exendin-4), 장 흡수(intestinal absorption)

## C. DOCUMENTS CONSIDERED TO BE RELEVANT

| Category* | Citation of document, with indication, where appropriate, of the relevant passages | Relevant to claim No. |
|---|---|---|
| X<br>Y | MADSEN, K. B. et al. Acute effects of continuous infusions of glucagon-like peptide (GLP)-1, GLP-2 and the combination (GLP-1 + GLP-2) on intestinal absorption in short bowel syndrome (SBS) patients. A placebo-controlled study. Regulatory Peptides. 2013, vol. 184, pp. 30-39 (online publication date: 16 March 2013).<br>See abstract; pages 2 and 31; and table 3. | 1-3,6,13,22<br>4,5,7-12,14-21 |
| Y | KR 10-2011-0110174 A (INDIANA UNIVERSITY RESEARCH AND TECHNOLOGY CORPORATION) 06 October 2011 (2011-10-06)<br>See paragraphs [0007]-[1361]. | 4,5,7,8,14-21 |
| Y | KR 10-2019-0037181 A (HANMI PHARM. CO., LTD.) 05 April 2019 (2019-04-05)<br>See claims 2 and 4; and paragraph [0039]. | 9-12 |
| A | JP 2014-058528 A (AMYLIN PHARMACEUTICALS LLC et al.) 03 April 2014 (2014-04-03)<br>See entire document. | 1-22 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 April 2022** | **12 April 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208**<br>Facsimile No. **+82-42-481-8578** | Telephone No. |

Form PCT/ISA/210 (second sheet) (July 2019)

**INTERNATIONAL SEARCH REPORT**

International application No.
**PCT/KR2021/019854**

**C. DOCUMENTS CONSIDERED TO BE RELEVANT**

| Category* | Citation of document, with indication, where appropriate, of the relevant passages | Relevant to claim No. |
|---|---|---|
| A | JP 2011-506442 A (CONJUCHEM BIOTECHNOLOGIES INC.) 03 March 2011 (2011-03-03) See entire document. | 1-22 |

Form PCT/ISA/210 (second sheet) (July 2019)

**INTERNATIONAL SEARCH REPORT**

International application No.
**PCT/KR2021/019854**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
**Information on patent family members**

International application No.
**PCT/KR2021/019854**

| Patent document cited in search report | Publication date (day/month/year) | Patent family member(s) | Publication date (day/month/year) |
|---|---|---|---|
| KR 10-2011-0110174 A | 06 October 2011 | AR 074811 A1 | 16 February 2011 |
| | | AU 2009-327418 A1 | 24 June 2010 |
| | | BR PI0922969 A2 | 24 September 2019 |
| | | CA 2747499 A1 | 24 June 2010 |
| | | CL 2011001498 A1 | 09 March 2012 |
| | | CN 102325539 A | 18 January 2012 |
| | | EP 2376097 A1 | 19 October 2011 |
| | | EP 3075385 A1 | 05 October 2016 |
| | | IL 213113 A | 31 July 2011 |
| | | IL 213113 B | 28 September 2017 |
| | | IL 213113 D0 | 31 July 2011 |
| | | JP 2012-512903 A | 07 June 2012 |
| | | JP 2015-180649 A | 15 October 2015 |
| | | MX 2011006524 A | 17 August 2011 |
| | | PE 03322012 A1 | 14 April 2012 |
| | | PE 20120332 A1 | 14 April 2012 |
| | | RU 2011129784 A | 27 January 2013 |
| | | RU 2550696 C2 | 10 May 2015 |
| | | SG 172291 A1 | 28 July 2011 |
| | | TW 201028162 A | 01 August 2010 |
| | | TW I489992 B | 01 July 2015 |
| | | US 2011-0288003 A1 | 24 November 2011 |
| | | US 8969288 B2 | 03 March 2015 |
| | | WO 2010-071807 A1 | 24 June 2010 |
| KR 10-2019-0037181 A | 05 April 2019 | AR 113258 A1 | 11 March 2020 |
| | | AU 2018-339210 A1 | 16 April 2020 |
| | | AU 2018-339210 A1 | 04 April 2019 |
| | | BR 112020006189 A2 | 13 October 2020 |
| | | CA 3084326 A1 | 04 April 2019 |
| | | CN 111433221 A | 17 July 2020 |
| | | EA 202090571 A1 | 30 July 2020 |
| | | EP 3689898 A1 | 05 August 2020 |
| | | IL 273528 D0 | 31 May 2020 |
| | | JP 2020-534840 A | 03 December 2020 |
| | | PH 12020550105 A1 | 07 December 2020 |
| | | SG 11202002563 A | 29 April 2020 |
| | | TW 201927806 A | 16 July 2019 |
| | | US 2020-0262888 A1 | 20 August 2020 |
| | | WO 2019-066586 A1 | 04 April 2019 |
| | | ZA 202001888 B | 26 May 2021 |
| JP 2014-058528 A | 03 April 2014 | AU 2007-284365 A1 | 21 February 2008 |
| | | AU 2007-284365 A2 | 16 July 2009 |
| | | AU 2007-284365 A2 | 21 February 2008 |
| | | BR PI0606992 A2 | 28 July 2009 |
| | | CA 2597649 A1 | 17 August 2006 |
| | | CA 2660835 A1 | 21 February 2008 |
| | | CN 101155828 A | 02 April 2008 |
| | | EA 011653 B1 | 28 April 2009 |
| | | EA 200701704 A1 | 28 February 2008 |
| | | EP 1853627 A2 | 14 November 2007 |

Form PCT/ISA/210 (patent family annex) (July 2019)

**INTERNATIONAL SEARCH REPORT**
**Information on patent family members**

International application No.
**PCT/KR2021/019854**

| Patent document cited in search report | Publication date (day/month/year) | Patent family member(s) | Publication date (day/month/year) |
|---|---|---|---|
| | | EP 2057188 A2 | 13 May 2009 |
| | | EP 2392595 A1 | 07 December 2011 |
| | | IL 185563 A | 06 January 2008 |
| | | IL 185563 D0 | 06 January 2008 |
| | | JP 5399244 B2 | 29 January 2014 |
| | | KR 10-2007-0115947 A | 06 December 2007 |
| | | MX 2007009760 A | 07 November 2007 |
| | | NO 20074545 A | 09 November 2007 |
| | | NO 20074545 L | 09 November 2007 |
| | | NZ 561361 A | 26 February 2010 |
| | | SG 159551 A1 | 30 March 2010 |
| | | US 2011-0136737 A1 | 09 June 2011 |
| | | US 2013-0137631 A1 | 30 May 2013 |
| | | US 2013-0196913 A1 | 01 August 2013 |
| | | US 2013-0281374 A1 | 24 October 2013 |
| | | WO 2006-086769 A2 | 17 August 2006 |
| | | WO 2006-086769 A3 | 02 November 2006 |
| | | WO 2008-021560 A2 | 21 February 2008 |
| | | WO 2008-021560 A3 | 10 July 2008 |
| JP 2011-506442 A | 03 March 2011 | CA 2708762 A1 | 18 June 2009 |
| | | CN 102026666 A | 20 April 2011 |
| | | CN 102026666 B | 16 October 2013 |
| | | EP 2231191 A2 | 29 September 2010 |
| | | US 2009-0186819 A1 | 23 July 2009 |
| | | WO 2009-075859 A2 | 18 June 2009 |
| | | WO 2009-075859 A3 | 28 January 2010 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5424286 A
- KR 1020190037181
- WO 9734631 A
- WO 9632478 A


### Non-patent literature cited in the description


- **LJUNGMANN K. et al.** *Am. J. Physiol. Gastrointest Liver Physiol.,* 2001, vol. 281 (3), G779-85
- **H. NEURATH ; R.L. HILL.** The Proteins. Academic Press, 1979